(19)

Europäisches
Patentamt

European
Patent Office

Office européen
des brevets

(11)    **EP 4 768 913 A1**

(12)    **EUROPEAN PATENT APPLICATION**

(43) Date of publication:
    **01.07.2026   Bulletin 2026/27**

(21) Application number: **24223788.1**

(22) Date of filing: **30.12.2024**

(51) International Patent Classification (IPC):
    **G01N 33/543** $^{(2006.01)}$     **G01N 33/569** $^{(2006.01)}$

(52) Cooperative Patent Classification (CPC):
    **G01N 33/54373; G01N 33/56911**

(84) Designated Contracting States:
    **AL AT BE BG CH CY CZ DE DK EE ES FI FR GB
    GR HR HU IE IS IT LI LT LU LV MC ME MK MT NL
    NO PL PT RO RS SE SI SK SM TR**
    Designated Extension States:
    **BA**
    Designated Validation States:
    **GE KH MA MD TN**

(71) Applicant: **imd BIOTECH GmbH
    1190 Wien (AT)**

(72) Inventors:
    • **Werzer, Thomas
      1190 Wien (AT)**
    • **Unger, Christine
      1190 Wien (AT)**

(74) Representative: **Sommer, Andrea et al
    Andrea Sommer
    Patentanwälte Partnerschaft mbB
    Uhlandstraße 2
    80336 München (DE)**

(54)    **METHOD FOR THE DETECTION OF ONE OR MORE MICROORGANISMS**

(57)    The present invention concerns a method for the detection of one or more microorganisms in a measuring cell that comprises sensors with detection elements immobilized thereon with a binding site that selectively binds to analytes. The cell medium is a culture medium that supports the growth of the microorganisms to be detected. The pH of the cell medium is controlled. The present invention also concerns a measuring device for use in the method of the present invention.

Figure 1

**Description**

**Introduction**

The present invention concerns a method for the detection of one or more microorganisms in a measuring cell that comprises sensors with detection elements immobilized thereon with a binding site that selectively binds to analytes. The cell medium is a culture medium that supports the growth of the microorganisms to be detected. The pH of the cell medium is controlled. The present invention also concerns a measuring device for use in the method of the present invention.

**Background of the invention**

The analysis of biological systems has proceeded at a fast pace in recent decades and is still one of the most active areas of research. The detection or determination of the concentration of a biological analyte is hampered by the complexity of biological systems as the targeted analysis of a single compound in a mixture of a large number of different compounds is a challenging task. Detection of the target analyte usually contains numerous steps including amplification, isolation, dilution or pre-concentration steps followed by a detection step. This does not only require a lot of manual labor from trained operators but is also more time consuming and prone to errors.

One method often used to isolate either antigens or antibodies from a complex matrix, e.g. a complex biological sample, for the following analysis are reactions of an antibody with an antigen. The ELISA method is representative for such a method.

In another method oscillating piezo-electric crystals are used for the analysis of biological systems. Shons et al. discloses in 1972 for the first time the usage of piezoelectric crystals and antigen-antibody reactions for the analysis of biological systems. The surface of a piezoelectric crystal was coated with bovine serum albumin (BSA) and a change in frequency was observed when the piezoelectric crystal was brought into contact with an antibody that bound to the BSA (A. Shons, F. Dorman, J. Najarian, J. Biomed. Mater. Res. 6:565). This method is named Quartz Crystal Microbalance (QCM), i.e. a method of weighing using a quartz piezoelectric crystal. The change in frequency follows in principle the equation $f_n - f_0$ = -constant $*$ $(m_n - m_0)$, wherein $f_0$ is the resonance frequency of the quartz piezoelectric crystal before addition of a mass (in the aforementioned example the mass of the antibodies), $f_n$ is the resonance frequency after addition of the mass, $m_0$ is the starting mass of the electrode and $m_n$ is the mass of the electrode after addition of a mass. The change in frequency and other mechanical properties, e.g. motional resistance, depends not only on the mass of the molecules bound, but also on their size, the viscoelastic properties and other properties of the molecules bound and on the properties of the medium.

Usually, a capture molecule that serves as detection element, is attached to the crystal and the change in frequency is measured when the capture molecule reacts with the molecule to be captured (analyte). The capture molecule may be something other than antibodies or antigens, for example proteins or saccharides or polysaccharides. DNA, RNA and the like may also be used and may hybridize with the complementary DNA sequences, RNA or the like. In principle any functionality that selectively reacts with the analyte may be used as capture molecule.

The use of a capture molecule that specifically binds to a microorganism or parts thereof or to molecules expressed by a microorganism and the like allows in principle to identify microorganisms in a complex sample and/or to measure it quantitatively. The use of QCM for the detection and quantitative determination of microorganisms has been studied to some extent. Given that the purpose of most studies is the measurement of the contamination of food items, the limit of detection (LOD) is an important factor. Most studies report their LOD from their diluted solutions which presumes that the stock solutions must exhibit a larger microorganism concentration than reported. However, the LOD of a diluted sample has only subordinate importance for an early detection, as it requires a high amount of microorganisms in the growth medium that must be then diluted to reduce the interfering effects of the matrix. As none of the state-of-the-art methods report the specific detection of certain microorganisms directly in the growth medium it must be assumed that this detection is not possible, or not accurate enough due to interferences of the medium. This renders these methods to endpoint measurements that rely on the growth of a sufficient amount of microorganisms in the growth medium that must later be diluted for measurement, whereby the growth is terminated.

Campbell et al. used piezoelectric-excited millimeter-sized cantilever (PEMC) sensors in combination with immobilized antibody specific to *Escherichia coli* (EC) O157:H7 to detect *E. coli* at 10 cell/mL in batch mode and 1 cell/L in flow mode (G.A. Campbell, R. Mutharasan, "A method of measuring Escherichia coli 0157:H7 at 1 cell/mL in 1 liter sample using antibody functionalized piezoelectric-excited millimeter-sized cantilever sensor", Environmental Science & Technology 41 (2007) 1668-1674. https://doi.org/10.1021/es061947p.). The authors showed attachment and detachment of *E. coli* to and from their sensor. While the detection system is reported to work well and to be reusable, the authors do not show if the attachment was specific or unspecific. Also, the experiment was made under highly diluted conditions starting from a stock concentration of $1 \times 10^9$ cells per ml, therefore a $1:10^9$ dilution in phosphate-buffered saline (PBS, 1 cell/mL) and respectively $1:10^{12}$ (1 cell/L) which means that the matrix was highly diluted in PBS and not a complex biological system and not a growth medium. In addition, the cells were previously (undisclosed length of time) killed by formaldehyde and may not have maintained their structure, which means that the detection could originate from intracellular biomarkers. This remains also unclear since the antibodies clonality and the antigen are undisclosed in this publication.

Also, quartz crystal microbalances were used as immunosensors for *Salmonella typhimurium* with simultaneous measurements (X.-L. Su, Y. Li, "A QCM immunosensor for Salmonella detection with simultaneous measurements of resonant frequency and motional resistance", Biosensors and Bioelectronics 21 (2005) 840-848. https://doi.org/10.1016/j.bios.2005.01.021). The coating comprised protein A for the immobilization of the antibody to the sensor surface (capture antibody, i.e. the detection element). S. typhimurium was detected in chicken meat samples in a concentration range of $10^5$ - $10^8$ cells/mL. After external amplification 100 cells/mL could be detected. External amplification comprised harvesting with immuno-magnetic nanoparticles that were then added to the sensor. No interference was observed from *E. coli* K12 or the sample matrix in the concentration ranges used for Salmonella. However, the best results in amplification could only be achieved in a flow cell by stopping the flow, incubating the amplifiers and flushing with PBS.

The same authors made a QCM immunosensor for the detection of *E. coli* O157:H7. The layer was made from a monolayer of 16-mercaptohexadecanoic acid (MHDA) and an antibody (X.-L. Su et al., "A self-assembled monolayer-based piezoelectric immunosensor for rapid detection of Escherichia coli O157:H7", Biosensors and Bioelectronics 19 (2004) 563-574. https://doi.org/10.1016/S0956-5663(03)00254-9). The authors investigated three procedures, immersion, dip-and-dry, and a flow-through method. In dip-and-dry, where the sensor was only dipped in different solutions of the bacterium, the lowest LOD of $10^3$ CFU/ml was obtained, all other methods resulted in higher LODs. However, the authors used an undisclosed concentration of bacteria in their stock solution. The dilution of the solution is therefore unknown. For this study, the authors worked with heat deactivated *E. coli.* No cross-reactivity analyses were made.

Ripa et al. used QCM to analyze bacterial growth from a $2 \times 10^7$ CFU/mL stock solution, without any coating using only the bare Gold or Titanium surface (R. Ripa et al, "Detecting Escherichia coli Biofilm Development Stages on Gold and Titanium by Quartz Crystal Microbalance", ACS Omega 5 (2020) 2295-2302. https://doi.org/10.1021/acsomega.9b03540). They were able to detect the bacterial growth within 24 h. However, without a selective layer the signals could originate from any biomolecules within the sample and it is not possible to differentiate between bacterial species, i.e. the measurement is not selective.

Vaughan et al. disclosed QCM immunosensors for the detection of *Listeria monocytogenes* made with a thiosalicylic acid self-assembling monolayer (SAM) and attached antibodies. The sensor was able to detect *L. monocytogenes* cells in real time in a solution of $1 \times 10^7$ cells/ml (Vaughan et al, "Development of a quartz crystal microbalance (QCM) immunosensor for the detection of Listeria monocytogenes", Enzyme and Microbial Technology 29 (2001) 635-638. https://doi.org/10.1016/S0141-0229(01)00449-5). The sensors were reported to be reusable for more than 10 times and were non-reactive against Bacillus cereus. However, the authors immobilized capture antibodies that were diluted in tris(hydroxymethyl)aminomethane (TRIS) buffer, which is known to quench N-Hydroxysuccinimide (NHS) ester surfaces, which were used in this study. The use of the TRIS buffer may have inhibited the covalent binding of the capture antibodies. The viability of the investigated bacteria, and purification, cultivation and dilution factor of the bacteria were not disclosed. The document does not disclose the use of a growth medium or any other complex biological system in the measuring cell.

Jiang et al used a signal amplification step to improve the QCM signals with (nano-)particles of different sizes and materials (magnetic, silica and polymer and diameters from 30 nm to 970 nm) and achieved a more sensitive detection of *Escherichia coli* O157:H7 (X. Jiang et al "Evaluation of different micro/nanobeads used as signal amplifiers in QCM immunosensor for more sensitive detection of E. coli O157:H7", Biosens. Bioelectron. 29 (2011) 23-28. https://doi.org/10.1016/j.bios.2011.07.059). Both the sensor and the particles were conjugated with anti-*E. coli* antibodies. Their method comprised the capture of *E. coli* O157:H7 cells to the sensor and a subsequent signal amplification by sandwiching micro/nanobeads labeled secondary antibodies. The broadest linearity range was achieved in a concentration range between $10^3$ and $10^6$ CFU/mL using magnetic nanobeads (350 nm) as signal amplifier. The cultures were heat killed before measurement and the cross-reactivity, which may be stronger when using nanobeads, was not investigated.

Kleo et al. studied *F. tularensis* bacteria with microfluidic QCM flow cells (K. Kleo et al., "Fast detection of pathogenic bacteria by using different sensor techniques", in: Proceedings IMCS 2012, Nürnberg/Nuremberg, Germany, AMA Service GmbH, Von-Münchhausen-Str. 49, 31515 Wunstorf, Germany, 5/20/2012 -5/23/2012, pp.834-836). Alternative sensor systems for these pathogenic bacteria are presented. Two different immunological detection systems for *F. tularensis* are demonstrated. The first technique is based on quartz crystal microbalance (QCM) which uses sensor chips modified by a specific antibody. A detection limit of $10^3$ CFU/ml can be obtained. Again, in this study the bacteria were inactivated before detection, which concludes that the measurement was not done during growth.

Pathogenic microorganisms are omnipresent in the environment and represent major risks for human and animal health. There are different pathways by which humans and animals can be infected and develop diseases that can be even fatal depending on the pathogenic microorganism. To treat the disease appropriately (e.g. by antibiotics) it is of paramount importance to identify the pathogen. Besides highly contaminated environments (e.g. hospitals, wastewater), one major pathway of getting in contact with pathogenic microorganisms is by contaminated food.

For food companies, contamination represents a major economic risk, as contaminated food must be recalled, production must be stopped and decontaminated, compensation must be paid to those affected, and the company suffers reputational damage. For these reasons, food is regularly tested for microorganisms by food companies and the authorities in many countries. For example, the official test for the pathogen *Listeria monocytogenes* and *Listeria spp.* according to EN ISO 11290-1:2017 includes a first enrichment (24 to 26 h; 30°C; half-Fraser broth), a second enrichment (24 h; 37°C; Fraser broth), and plating of samples from the first and second enrichment on agar plates with two different media, with incubations between 24 to 48 h each and then the colonies being counted and detected using molecular biological techniques. The test quantity comprises 25 g for solid or 25 ml for liquid samples in 225 g or ml (1:10) of the first enrichment medium. For the second enrichment, 0.1 ml of the first enrichment medium is added to 10 ml of the second medium. The duration of the test according to EN ISO 11290-1:2017 is therefore at least 72 h.

In addition to the legally required testing, food manufacturers carry out so-called rapid tests, which are based on PCR, ELISA or similar methods and can be carried out more quickly than the method according to EN ISO 11290-1:2017. In addition to testing the food items themselves, these methods are also used for so-called environmental samples in order to detect contamination in and around the production facilities at an early stage and to prevent transmission to food at an early stage. The rapid tests are usually used after the first enrichment and can identify microorganisms that are sampled after 22 hours at the earliest (e.g.: 3M PCR operating instructions).

A disadvantage of most rapid test methods is that for the measurement a sample must be processed and the measurement represents only one point in time. Typically, the sampling and/or measurement is made after pre-enrichment for a fixed time interval (typically 24 hours), to ensure that the microorganism concentration is high enough to yield an accurate measurement result, which renders the methods to endpoint measurements, because enrichment stops necessarily with the measurement. Endpoint measurements have disadvantages, as very small contaminations or delayed growth of the microorganisms (lag phase) can lead to a microorganism concentration below the detection limit and make another measurement necessary at a later point in time. On the other hand, higher microorganism contaminations, which could have been detected earlier, are measured after the same enrichment and analysis time. Usually, enrichment steps are necessary to increase the number of bacteria to a level that is above the detection limits of the method of choice after enrichment and before measurement growth is terminated. If the microorganism concentration is high in the initial sample, a detection could be possible earlier, as the bacteria would reach numbers above the detection limits earlier. However, in an endpoint method, an earlier detection is not possible, as the protocol foresees an incubation for the predefined time, to ensure that also all concentrations of microorganisms are detected, including small concentrations.

In summary, all known methods are either reliant on at least one pre-enrichment step and/or multiple sample handling and/or processing and/or preparation steps. There is no simple method that combines microorganism enrichment and the selective or specific detection of microorganism growth in one step as these methods either need sample processing steps or suffer from interferences with complex matrices that require sample dilution. Furthermore, most known methods cannot detect the viability of microorganisms and require an additional confirmation of the viability by further tests.

There remains a need for a method to selectively detect microorganisms directly in a complex biological sample and to measure the microorganism quantitatively. Furthermore, there remains a need for a method to determine if the microorganisms are dead (or not culturable) or alive and can proliferate. Furthermore, these tasks should be performed in a time efficient manner and with a procedure as simple as possible, reducing multiple sample preparation and/or processing and/or handling steps.

**Purpose of the present invention**

It is the purpose of the present invention to provide a measuring device and/or a measuring method that allows for improvement in the detection and/or quantitative measurement of microorganisms and avoids the aforementioned problems of the prior art. One purpose of the present invention is to provide a measuring device and/or a measuring method that allows the selective and/or specific detection and/or quantitative measurement of microorganisms in complex matrices, e.g. in samples comprising or consisting of complex biological material. It is especially one purpose of the present invention to provide a measuring device and a measuring method that allows the selective and/or specific detection and/or quantitative measurement of microorganisms in the presence of other microorganisms. It is also a purpose of the present invention to provide a measuring device and a measuring method that allows the selective and/or specific detection and/or quantitative measurement of microorganisms during enrichment, especially in complex matrices that comprise other microorganisms and/or other impurities. It is further a purpose of the present invention, to provide a measuring device and a measuring method that allows to determine the proliferation of microorganisms. Further, it is desired that the aforementioned intended purposes are performed in a very short time, e.g. preferably 48 hours or less.

In addition, it is desired that apparatus and method allow for a simple measurement procedure to determine the presence of target microorganisms. This especially includes that the sample preparation should be as simple as possible. Measurement should be possible for pathogenic and non-pathogenic microorganisms.

**Detailed description**

Measuring Device

In one aspect the present invention concerns a measuring device for the detection of one or more microorganisms, the measuring device comprising a measuring cell, one or more sensors and a cell medium, wherein

- the measuring cell comprises the one or more sensors and the cell medium,

- one or more detection elements are immobilized on at least one part of a surface of each of the one or more sensors,

- the one or more detection elements are in contact with the cell medium,

- the at least one or more detection elements comprise one or more binding sites that selectively bind to one or more analytes, wherein the one or more analytes may be any substance that is an indicator for the presence of the one or more microorganisms,

- the one or more sensors are able to detect the binding of at least one of the one or more analytes to the one or more sensors,
characterized in that

- the cell medium is a culture medium that supports the growth of the microorganisms to be detected and in that

- the measuring device comprises means to control the pH value of the cell medium.

The measuring device of the present invention allows the selective detection and/or the selective quantitative measurement of microorganisms in complex biological samples, i.e. in the presence of growth medium and/ or in the presence of other microorganism and/or in the presence of other biological organic and inorganic contents of the biological sample. Growth medium and impurities, such as other microorganisms, other biological organic and inorganic contents of the biological sample contaminate the cell medium and can make accurate measurements difficult or impossible. The interference of such ingredients with the measurement may be the result of deposition of material on the sensor or by other effects. The present invention allows for selective and accurate detection and quantitative measurements of microorganisms because the cell medium does not interfere with the measurement. It also allows to perform the measurements in short time and in a continuous measurement, because the sample is not destroyed during measurement, as is the case for example of the method according to EN ISO 11290-1:2017, ELISA and PCR measurements. This is of extreme importance, as it does not only have the potential to accelerate the detection of microorganisms, especially in industrial settings, but also simplifies the measurement. Non-continuous measurements usually rely on a fixed time for enrichment, e.g. by cultivating, before the sample is measured. That is, even if a microorganism is enriched sufficiently in culture before the end of the fixed time, so that it could be detected before the end of the fixed time, the measurement is only taken after the fixed time has ended and the information that the microorganism is present is only received after the fixed time has ended. The waiting time for the measurement result is unnecessarily long. This problem may potentially be solved by taking several samples that are measured after different time intervals. But it is evident that this results in additional outlay and is not meaningful for industrial purposes. The continuous measurement is discussed further below in the context of the method of the present invention. The measuring device of the present invention comprises (1) a measuring device that allows for the selective detection and/or selective quantitative measurement of microorganisms and (2) a cell medium that is a culture medium that supports the growth of the microorganisms to be detected or quantitatively measured or both.
In addition, measuring device and method of the present invention allow for a simple measurement procedure that requires only the addition of the sample or an addition of growth medium followed by sample addition (or vice versa) or an addition of growth medium with sample to determine the presence of target microorganisms.

Definitions

Selective in the meaning of the present invention relates to the detection of a single microorganism or of a group of microorganisms, whereby other microorganisms are not detected. Selectivity to a single microorganism is herein also designated the term "specific", e.g. a selective detection of a single microorganism may be designated a "specific detection."
The term "detection of one or more microorganisms" in the present invention includes the qualitative detection of the microorganism, i.e. the mere confirmation of its presence in the measuring cell and the quantitative measurement of the amount of the microorganisms in the measuring cell. In a preferred embodiment the measuring device is a measuring device for the quantitative measurement of the amount of one or more microorganisms. The terms "growth medium" and "culture medium" are used interchangeably herein.

The cell medium is a liquid aqueous medium. The term "cell medium" refers to the whole liquid content of the measuring cell.

pH value, Buffer

As stated above, the present invention is characterized in that the cell medium does not interfere with the measurement. For this purpose, the measuring device of the present invention comprises means to control the pH value of the cell medium. Surprisingly, controlling the pH value reduces or completely suppresses interference of other ingredients of the cell medium than the microorganism to be measured. Without wishing to being bound by theory, the inventors believe that interference of the cell medium with the sensor may be the result of uncontrolled deposition or even adsorption of ingredients of the cell medium on the sensor and possibly as well on other parts of the measuring cell. The cell medium comprises the ingredients of a growth medium, e.g. amino acids, proteins, salts and the like. In addition, as a result of the cultivation of the sample it may comprise other microorganisms than the ones to be measures. It may also comprise compounds expressed by microorganisms therein and other compounds present in the sample to be measured. The solubility of amino acids, proteins, microorganisms and other ingredients of the cell medium may be dependent on the pH. The same may be true for the interaction of these compounds with the sensor surface. Most microorganisms change the pH of the medium during cultivation. They mostly lower the pH. The pH may also change for other reasons, i.e. during addition of the sample or other ingredients to the cell medium or dilution or for any other reason. In any case, it is shown in the example of the present invention that controlling the pH will suppress unspecific depositions on the sensor.

The pH must be controlled in a range that does not produce unspecific deposition on the sensor. An unspecific deposition is any deposition on or any binding to the sensor than that of the analyte(s) to be detected. The necessary range of the pH value may be determined by simple preliminary experiments, in which a sensor that does not comprise a detection element is brought into contact with a cell medium with a certain pH. Such a sensor may for example be prepared by the procedures described in the chapter on sensor preparation and in comparative example 1 in the experimental part. The measurement is otherwise performed as intended for the detection of a microorganism or microorganisms and the pH value is changed to various pH values, for example over the range of for example 5 to 9. If the sensor produces a signal, the pH value at which the signal is obtained must be avoided.

Preferably the pH of the cell medium is controlled in the range of 5 to 9, more preferably in the range of 6 to 8.5 and most preferably in the range of 6 to 8. In an especially preferred embodiment, the pH is controlled within a pH value range, preferably in a range of 2 pH value units, more preferably within a range of 1 pH value unit, even more preferably within a range of 0.6 pH value units and most preferable with in a range of 0.2 pH value units.

In a further preferred embodiment, the pH value is at any time during measurement in the range of 5 to 9 and pH is controlled within a pH value range therein of 2 pH value units. more preferably within a range of 1 pH value unit, even more preferably within a range of 0.6 pH value units and most preferable with in a range of 0.2 pH value units.

In a further preferred embodiment, the pH value is at any time during measurement in the range of 6 to 8.5 and pH is controlled within a pH value range therein of 2 pH value units more preferably within a range of 1 pH value unit, even more preferably within a range of 0.6 pH value units and most preferable with in a range of 0.2 pH value units.

In a further preferred embodiment, the pH value is at any time during measurement in the range of 6 to 8 and the pH is controlled within a pH value range therein of 1 pH value unit, more preferably within a range of 0.6 pH value units and most preferable with in a range of 0.2 pH value units.

Any known means to regulate the pH value is usable in the present invention. In one embodiment the measuring device may comprise a pH meter and means to add acids and/or bases to the cell medium to regulate its pH value. If a buffer is used, the capacity of the buffer of the buffered culture medium is selected such that the pH value of the cell medium is controlled in one of the aforementioned ranges.

In an especially preferred embodiment, the cell medium is buffered. This is an especially simple and effective means to control the pH value of the cell medium. This reduces or completely suppresses interference of the cell medium with the measurement. Without wishing to be bound by theory, the inventors are of the opinions that when buffers are used, in addition to the effect of the control of the pH value, as a minor effect, the buffer may also inhibit unspecific depositions because of the increased ionic strength of the cell medium. As buffer, any buffer may be used that is known to the expert in the art. The buffer may for example be selected from the group consisting of phosphate buffer, 2-(N-morpholino) ethanesulfonic acid (MES) buffer, tris(hydroxymethyl)aminomethane (TRIS) buffer, 4-(2-hydroxyethyl)-1-piperazineethanesulfonic acid (HEPES) buffer, (N-morpholino)propanesulfonic acid (MOPS) buffer and citrate buffer.

The pH value of the cell medium is preferably set to a predetermined value. Preferably in the aforementioned ranges. More preferably to a value in the range of 7.0 to 7.8 and most preferably to a pH value of 7.5. This value may depend on the analyte and/or the composition of the cell medium. This predetermined value may also be determined by simple preliminary test, in which a cell medium to be measured is exposed to various pH values in the measuring cell of the measuring device of the present invention, but without a detection element, as described above, under measurement conditions and a pH value is determined at which no deposition on the sensor takes place, i.e. no signal is produced.

Preferably the pH during the measurement is controlled such that it is in a range of +-1.0 pH value unit of the predetermined value during the measurement. More preferably the pH of the cell medium is kept during the measurement in a range of +- 0.5 pH value units of the predetermined value during the measurement. Even more preferably the aforementioned range is +- 0.3 pH value units and most preferably +- 0.1 pH value units. If the pH value is kept in the aforementioned ranges, the interference is reduced effectively and therefore the accuracy of the measurement is further improved.

If a buffer is used, the capacity of the buffer of the buffered culture medium is selected such that the pH value of the cell medium is controlled in one of the aforementioned ranges around the predetermined value. The necessary capacity of the buffer to avoid major fluctuations in the pH value can be determined by simple preliminary tests, by simply performing the measurement and measuring the pH value. If the pH value fluctuates too much, the concentration of the buffer must be increased. It can also be determined by the aforementioned test wherein a sensor without detection element is used and the measurement is otherwise performed under normal measurement condition. The necessary capacity of the buffer cannot be stated for all embodiments of the present invention, as it depends on the buffer and the composition of the cell medium, including the growth medium comprises therein and the microorganisms and the sample comprised therein. It can however be simply be determined for any measurement as described above. As can be seen from the examples of the present invention, 10xPBS, i.e. a 0.1 M phosphate buffer which is additionally 1.4 M in NaCl and 0.027 M in KCl was sufficient for all experiments disclosed in the experimental part of the present invention. A phosphate buffer that is 0.1 M or above should therefore be sufficient for most measurements. If preliminary measurements show that such a buffer is not sufficient for a certain application, the buffer strength should be increased. Preferred is a 0.05 M phosphate buffer or above. There is not upper limit for the concentration of a phosphate buffer in the cell medium of the present invention. Therefore a concentration of 0.1 M or more is preferred. For practical purposes an upper limit of 10 M is preferred. Preferred are 0.05 to 10 M more preferably 0.1 to 5 M.

As discussed above, interfering, especially nonspecific adsorption of contaminants of the cell medium on the sensor leads to erroneous measurements or makes selective detection and/or quantitative measurement impossible or even makes any measurement impossible. If such interfering of the cell medium with the sensor is avoided, measurements may be performed with any biological sample. Dilution of a biological sample in order to suppress interference of the contaminants of the sample with the sensor is not necessary any more or only necessary to a lesser extent. In addition, measurements with biosensors in such buffers can be performed in a continuous fashion allowing the continuous detection of microorganisms at any time during the measurement.

As discussed above, the pH value of the cell medium is controlled for example by addition of acids and/or bases and preferably by a buffer. Since the cell medium is a growth medium, addition of the acids, bases or a buffer should preferably dilute the cell medium as little as possible, if acids, bases or buffer are added to a growth medium that already has all necessary ingredients comprises therein, the addition should preferably dilute the growth medium as little as possible, in order to keep the concentrations of the ingredients of the growth medium in the optimal ranges. Obviously, the cell medium may also be prepared from its basic ingredients and the concentrations of all ingredients including acids, bases and buffers may then be adjusted as necessary.

Sensor

The one or more sensors of the present invention may be any sensor that is able to detect a microorganism, but preferably at least one of the one or more sensors is a biosensor. A biosensor may provide specificity for certain microorganisms or a certain microorganism to the measuring device of the present invention. The sensors of the present invention are therefore preferably selective sensors, i.e. they can selectively detect and/or measure a single microorganism or a certain group of microorganisms, e.g. all microorganisms of a genus or all microorganisms with any other common property. More preferably they are specific sensors, i.e. they are able to selectively detect a single species of a microorganism. The one or more sensors of the present invention may be any type of sensor that allows measurement of microorganisms, but preferably the one or more sensors are independently selected from the list consisting of a piezoelectric sensor, a surface acoustic wave sensor or a surface plasmon resonance sensor. Such sensors are known and reliable sensors for the measurement of microorganisms. Preferably, the sensors are capable of measuring continuously. Preferably, the one or more sensors are piezoelectric sensors and more preferably quartz crystal microbalances (QCMs), i.e. a piezoelectric sensor that is made of quartz. Such sensors are known, reliable and are very sensitive. In a more preferred embodiment, the one or more sensor is a quartz crystal microbalance with an AT-cut quartz. The sensors and the sensor material may also have a more complex composition than just a single material. The oscillating frequency of piezoelectric sensors used in the present invention is preferably in the range of 1 to 150 MHz, more preferably in the range of 5 to 20 MHz and most preferably in the range of 6 to 10 MHz This is especially true for quartz crystal microbalances.

Coating of Sensor

The one or more sensors may be used as such or preferably at least one of them has a coating on its surface. Coatings for sensors are known and widely used in the art and provide the surface of the sensor with properties that the sensor material itself does not possess. Among other functions the coating may protect the surface of the sensor from the biological materials in the sample to be measured. It may also provide the sensor with a surface that binds the analyte. It may also provide specific functionality to bind reagents that allow further functionalization of the surface of the sensor, e.g. with linker (see below). Preferably the sensor has metal coating on its surface, more preferably a metal or an alloy that is not corrosive in contact with the sample to be measured. Therefore, preferably the coating is a noble metal and more preferably the surface of the sensor is coated with gold or silver. Most preferably it is coated with gold. Gold is inert and at the same time binds well with known linkers. Furthermore, linkers can be bound to a gold surface by simple procedures.

Adhesive Layer

In addition to the coating, there may be provided an adhesive layer between the coating and the surface of the blank sensor material. This coating may provide a better adhesion of the coating to the surface of the layer and/or may support the coating of the sensor with the aforementioned coating. It may also enhance other properties of the sensor. Such coatings are well known in the art. The adhesive layer preferably consists of a metal or a metal alloy and is more preferably selected from the group consisting of chromium, titanium or an alloy wherein the sum of the mass of chrome and titanium in the alloy is at least 50% by mass.

Linker

The sensor may also have one or more linker. A linker is located between the sensor and the detection element and binds the detection element to the sensor. Linkers for this purpose are known in the art and any known linker may be used in the present invention. The one or more linker may be bound directly to the surface of the sensor material or it may be bound to the aforementioned coating of the sensor. The one or more linker may be organic or inorganic. Preferably the linker is organic and more preferably a thiol linker or an anti-fouling coating. Most preferably the linker is a thiol linker. The linker may form a coating, i.e. may be a dense application of the linker on the sensor or the linker may leave some space between the linker molecules attached to the sensor, where the cell medium may come into direct contact with the sensor. The linker may also form a coating on parts of the sensor and not form a coating on other parts thereof. In preferred embodiments the linker forms a coating. As linker one compound or a plurality of compounds may be used.

As stated above, any known linker may be used. The linker has at least two functional groups a first functional group, which is able to bind to the surface of the sensor and a second functional group, which is able to bind to the detection element. The linker may have more than two functional groups. He may also have more than two kinds of functional groups. The first and the second functional group may also be a functional group that is able to bind to the surface of the sensor and be able to bind to the detection element. In this case the first functional group and the second functional group may be identical.

The first functional group is preferably selected from the group consisting of carboxyl, hydroxyl, primary amine, secondary amine and thiol. Most preferably, the first functional group is a thiol group. Thiols are preferable for sensors that have a surface that comprises noble metals or consist of noble metals, especially silver or gold, because thiol groups bind to these metals easily, when brought in contact therewith. Thiols are especially preferred for sensors that have a coating that comprises gold and most preferred for sensors that have a coating that consist of gold. In addition, organic thiols form self-assembled monolayers (SAMs) on noble metals, which are easily prepared and impede unwanted direct interaction of the cell medium with the sensor surface (e.g. anti-fouling). Such interaction may otherwise have a detrimental effect on the accuracy of the measurement.

The second functional group may be any group that can be used to bind the linker to the detection element. The second functional group is preferably selected from the group consisting of carboxyl, hydroxyl, primary or secondary amine, thiol and proteins. This is especially true for linker in which the first functional group is a thiol group.

If the linker is an organic compound, it preferably has the structure of Formula (I)

$$F^1-Hydr-F^2, \text{ Formula (I)}$$

wherein F1 and F2 are the first and second functional group and Hydr is a hydrocarbon group or a hydrocarbon group in which a part of the carbon atoms are substituted by hetero atoms. The hydrocarbon group may be saturated or unsaturated, straight or branched alkyl, aryl, aralkyl or heteroalkyl. Preferably the carbohydrate part thereof is a saturated straight alkylene residue, more preferably a saturated straight C1 to C30 alkylene residue and most preferably a saturated straight C2 to C20 alkylene residue.

In embodiments in which Hydr is a hydrocarbon group in which a part of the carbon atoms is substituted by hetero atoms, these heteroatoms are not part of a functional group. In this embodiment Hydr may be selected from the group consisting of ether groups, thioether groups or tertiary amine groups. This is especially true for linker in which the first functional group is a thiol group.

Preferred embodiment of thiols which comprise an additional functional group are as follows:

Carboxyl groups

Thiols which have at least one carboxyl group as second functional group are preferably omega-mercaptocarboxylic acids. More preferably straight chain C2 to C30 omega-mercaptocarboxylic acids, even more preferably straight chain C5-C20 omega-mercapto carboxylic acids and most preferably they are selected from the group consisting of n-11-mercaptoundecanoic acid and n-16-mercaptohexadecanoic acid.

Hydroxyl groups

Thiols which have at least one hydroxyl group as second functional group are preferably omega-mercaptoalcohols. More preferably saturated straight chain C2 to C30 omega-mercapto-1-alcohols, even more preferably straight chain C5-C20 omega-mercapto-1-alcohols and most preferably they are selected from the group consisting of n-6-mercapto-1-hexanol, n-8-mercapto-1-octanol and n-11-mercapto-1-undecanol.

Amino groups

Thiols which have at least one amino group as second functional group are preferably omega-aminothiols. More preferably saturated straight chain C2 to C30 omega- amino-1-thiols, even more preferably straight chain C2-C20 omega-amino-1-thiols and most preferred is cysteamine.

Thiol groups

Thiols which have at least one additional thiol group as second functional group are preferably 1,omega-alkyldithiols. More preferably saturated straight chain C2 to C30 1,omega-alkyldithiols, even more preferably saturated straight chain C2-C20 1, omega-alkyldithiols and are most preferably selected form the group consisting of 1,5-pentanedithiol and 1,8-octanedithiol.

Also preferred are thiophenols with at least one second functional group as described above. One example is thiosalicylic acid. However, thiosalicylic acid is preferably not used. Thiols that have more than one second functional group may also be used. A preferred example is 6,8-dithiol-n-octanoic acid. This compound maybe prepared in situ by oxidation of alpha-lipoic acid. Another preferred group of polyfunctional linkers are polythiols with more than two thiol groups, e.g. trithiols or tetrathiols and so on. Here the second functional group is also a thiol group. Another preferred group of polyfunctional linkers are saccharides, more preferably polysaccharides, even more preferably glucans and most preferably dextran.

No functional group

The aforementioned linker in which the first functional group is thiol may be used as linker together with thiols that do not have an additional functional group that reacts either with the surface of the sensor or with the detection element. Preferred examples for such thiols are monothiols, preferably saturated straight chain C1 to C30 monothiols, more preferably saturated straight chain C5 to C20 monothiols and most preferably 1-octanethiol. Such thiols serve to adjust the number of linkers on the surface of the sensor. Preferably the molar ratio of the thiols with at least one additional functional group to the thiols that do not have an additional functional group that reacts either with the surface of the sensor or with the detection element is in the range of 1 to 8 and more preferably in the range of 2 to 6. These shorter linkers align the layer better than longer linkers and fill it out. This is especially true for sensors with a gold coating.

A preferred embodiment of linkers in which the carbohydrate part of the linker comprises heteroatoms are polyoxyalkylenes of the formula $HS(-(CH_2-)m-O)n-X$, with m is 2 or 3 and preferably 2, n is preferably in the range from 2 to 5 and X is a second functional group as described above. It is known that such compounds form self-assembled monolayers that have antifouling properties, i.e. they prevent deposition of components of biological sample on the surface of the sensor. Among these compounds polyethylene glycols are preferred. Representative examples of such polyethylene glycols are:

HS-PEGn-OH (hydroxy), e.g. 1 to 2 kDa

HS-PEGn-COOH (carboxyl), e.g. 2 to 4 kDa, especially 2, 3.4 or 4 kDa

mPEGn-SH (methyl), e.g. 350 to 750 kDa

HS-PEGn-NH$_2$ (amine), e.g. 1 to 4 kDa, especially 2, 3.4 kDa

Herein n may be selected in a wide range. It is preferably selected such that the weight average molecular weight of the linker is in a range of 100 Da to 1.000 kDa. Preferred values for the molecular weight are given above for each linker individually.

In a preferred embodiment of the present invention the sensor is a quartz crystal microbalance with a gold coating and a linker coating on top of the gold coating that is a self-assembled monolayers comprising at least one monothiol and at least one omega-mercaptocarboxylic acid.

Detection Element

As stated above, the detection element comprises one or more binding sites that specifically binds to at least one of the one or more analytes. The binding site that specifically binds to an analyte can be anything that binds specifically to the analyte to be detected or measured. A variety of detection elements are known in the art and any of the known detection elements may be used in the present invention. A sensor of the present invention may be provided with one kind of detection element or it may be provided with more than one kind of detection elements. When more than one kind of detection element is used, the different kinds of detection elements may be selective or specific for the same microorganism or microorganisms or they may be selective or specific for the different microorganism or microorganisms.

The detection element may comprise or consist of one or more of item selected from the list consisting of proteins, DNA, RNA, antibodies or parts thereof, aptamers, lectins, metal organic frameworks, drugs, toxins, polymers and cells. As antibodies, monoclonal and polyclonal antibodies and other recombinant antibodies and parts thereof may be used. As polymers, for example molecularly imprinted polymers (MIPs), including nano-MIPS may be used. The detection element or a part thereof may even be a microorganism itself, for example when the analyte is an antibody that is expressed from the microorganism to be measured and the antigen of that antibody is a microorganism.

Antibodies that may be used as detection element may be polyclonal antibodies or monoclonal antibodies. The use of monoclonal antibodies is preferred when small molecules such as metabolites are the analyte. Polyclonal antibodies are preferred for the detection of more than one microorganism like a plurality of strains of a species or all strains thereof or a plurality of species of a genus or all species thereof. The antibodies may be antibodies whose antigen is any microorganism to be measured and more preferably they are selected from the list consisting of antibodies whose antigen is *E. coli,* antibodies whose antigen is a species of the genus *Listeria* and antibodies whose antigen is *L. monocytogenes.* The antibodies may also be antibodies whose antigen may be any substance that is an indicator for the presence of the one or more microorganisms and more preferably the antibodies whose antigen is any substance that is an indicator for the presence of a microorganism selected from the list consisting of *E. coli,* species of the genus *Listeria* and *L. monocytogenes*

In a preferred embodiment the compound that specifically binds to an analyte is a polymer. In a more preferred embodiment, the compound that specifically binds to an analyte comprised in the detection element is an antibody or a part thereof. Antibodies and parts therefore are easily assessable molecules that are highly selective for microorganisms and other substances. In a most preferred embodiment, the detection element comprises an antibody or is an antibody. It is also preferred that the detection element comprises or is a nanobody.

In preferred embodiments the detection element comprises in addition to the at least one compound that specifically binds to an analyte a group that allows binding of the detection element to the sensor, e.g. the linker. This may be a functional group, a linker or any other means known in the art for this purpose, e.g. a biotin group. The detection element may either be bound to the sensor itself, without any coating, it may also be bound to the coating or it may be bound to the linker. The latter is preferred.

The detection element may have in addition to the part of the molecule that comprises or is the binding site a second part that serves to connect the detection element to the sensor, preferably to the linker. This part may be anything that allows binding of the binding site to the sensor. This part may be for example the Fc regions or Fab regions of antibodies or they may be artificially bound to the binding site. In a preferred embodiment the detection element comprises proteins that serve to bind the linkers to compounds that comprise the one or more binding sites that selectively bind to at least one of the one or more analytes. These proteins are designate in the following as "binding proteins." These binding proteins are preferably able to form one or more non-covalent bonds with an antibody or they are able to easily form one or more covalent bonds to a molecule that comprises the binding site. Such molecules may for example easily bind to such protein via a biotin group. The binding proteins are preferably selected from the group consisting of protein A, protein G, protein A/G and other recombinant proteins that may form non-covalent bonds with an antibody. Protein A, protein G, protein A/G form non-covalent bonds preferably with the Fc region of antibodies. The antigen binding site of the antibodies is therefore free to bind to the analyte, i.e. the antigen. Most preferred the binding protein is protein A. A binding protein that is able to bind to biotin is preferably streptavidin. Therefore, the binding proteins are preferably selected from the group consisting of protein A, protein G, protein A/G and streptavidin. This is especially true for embodiments where the first functional group of a linker used is thiol. Such binding proteins provide the sensor with a high flexibility. A sensor may be prepared with a binding protein on the end of a linker, which is able to bind to the Fc region of antibodies. Such a sensor is not selective or specific, since it lacks the binding site for binding with a microorganism. It may be used in the measurement of any microorganism by simply adding an antibody into the measuring cell for the microorganism to be detected. The antibody binds in situ to the protein and to the microorganism and produces a sensor signal for the antigen of the antibody. If a different microorganism is to be detected, a different antibody may be added. Also, a plurality of antibodies may be added to detect a plurality of microorganisms. Such a sensor is therefore a generic sensor that may be used for the binding of any antibody or any group of antibodies and therefore may be used for the detection of various microorganisms as shown in the examples. Such a sensor is therefore especially preferred as it provides the measuring device and the method of the present invention with a high flexibility.

For embodiments comprising a binding protein, the linker comprises preferably any of the aforementioned linkers, wherein the second functional group is a carboxyl group. More preferably combined structure of linker and binding protein has the structure of Formula (II):

HS-Hydr-CO-BProt, Formula (II),

wherein Hydr is as described above for the group Hydr in Formula (I) and BProt is a binding protein as described above. For the group Hydr all the preferred embodiments as described above for Formula (I) also apply to Formula (II). In addition, for the partial structure HS-Hydr-CO- the preferred embodiments stated above for linkers with thiol groups that have as second functional group a carboxyl group and the preferred embodiment for linkers of the formula HS-PEGn-COOH apply mutatis mutandis to this partial structure of Formula (II).

In a preferred embodiment of the present invention the sensor is a quartz crystal microbalance with a gold coating and a linker coating on top of the gold coating that is a self-assembled monolayers comprising at least one compound according to Formula (II). In a preferred embodiment of the present invention the sensor is a quartz crystal microbalance with a gold coating and a linker coating on top of the gold coating that is a self-assembled monolayers comprising at least one monothiol and at least one omega-mercaptocarboxylic acid, wherein the detection element comprises at least one antibody.

Signal Amplifier

In a preferred embodiment the cell medium comprises one or more signal amplifiers. That is in all embodiment of the cell medium comprised herein, the cell medium may comprise signal amplifiers, unless explicitly stated otherwise or unless it is evident from the context that the cell medium does not comprise signal amplifiers. Signal amplifiers are known in the art and any known signal amplifier that is suitable for the detection and/or measurement of the targeted microorganism may be used in the present invention. The signal amplifiers are preferably added to the cell medium. In the case of QCM sensors signal amplifiers are usually used to increase the molecular weight of the analyte by forming an analyte-signal amplifier complex. A higher molecular weight leads to a higher change in resonance frequency of the quartz sensor and therefore results in a more pronounced signal and an improved LOD. For this purpose, the signal amplifier binds to the analyte. The signal amplifier may be any substance that can bind to the analyte. Both the detection element and the signal amplifier bind to the analyte. They may be the same or different and they may comprise the same or a different binding site that specifically binds to at least one of the one or more analyte. Both the detection element and the signal amplifier may for example independently be selected from the group consisting of proteins, DNA, RNA, antibodies or a part thereof, aptamers, lectins, metal organic frameworks, drugs, toxins, polymers and cells. As antibodies, monoclonal and polyclonal antibodies and other recombinant antibodies may be used. As polymers, for example molecularly imprinted polymers (MIPs), including nano-MIPS may be used. The choice of the signal amplifier may depend on its ability to bind to the analyte and/or the molecular weight of the signal amplifier. In general, the higher the molecular weight, the better. In a preferred embodiment the signal amplifier is selected from the group consisting of proteins, DNA and RNA. In a more preferred embodiment the signal amplifier is an antibody or a part thereof and most preferred signal amplifier is an antibody. Antibodies that bind to a microorganism are usually readily available for most microorganisms, i.e. they are either commercially available or their preparation is described in the prior art. If not, they may also be prepared by use of the analyte as antigen by known methods. In a further preferred embodiment, the signal amplifier is additionally bound to a substance that increases the mass of the signal amplifier. This again serves to increase the mass of the analyte even more and therefore the LOD of QCM sensors and other sensors that measure the molecular weight of the analyte are improved. As substance that increases the mass of the signal amplifier preferably nanoparticles or nanobodies are used. Substances that increase the mass of the signal amplifier and especially nanoparticles for that purpose are well known in the art. The signal amplifier may therefore be an antibody bound to a nanoparticle. As nanoparticles any nanoparticle may be used, but preferably the nanoparticle is selected from the group consisting of nanoparticles that comprise or consist of gold, silver and silica. In a more preferred embodiment, the nanoparticles or nanobodies are bound to antibodies or MIPs. Most preferred the signal amplifier is an antibody to which a nanoparticle or a nanobody is bound.

More than one kind of signal amplifier may be used, e.g. more than one kind of antibody. Furthermore, the analyte may be bound to one or more signal amplifiers and a plurality of signal amplifiers that bind to an analyte may be all the same amplifiers or may be different kinds of amplifiers. For example, an analyte may be a microorganism and this microorganism may be bound to a plurality of molecules of the same kind of antibody or the microorganism may be bound to a plurality of molecules of two or more antibodies.

The signal amplifier may be bound to one or more molecules of the substance that increases the mass of the signal amplifier. If bound to more than one substance that increases the mass of the signal amplifier, it may be bound to a plurality of the same substance that increases the mass of the signal amplifier or it may be bound to a plurality of different substances that increase the mass of the signal amplifier. The signal amplifier may also be a substrate to which more than one group that may form an analyte-signal amplifier complex are bound. These groups may be selected as stated above for the signal amplifier from the group consisting of proteins, DNA, RNA, antibodies or a part thereof, aptamers, lectins, metal organic frameworks, drugs, toxins, polymers and cells. The substrate may for example be a nanoparticle or a nanobody as stated above.

The signal amplifier is usually added in an amount of 1 to 200 $\mu$g/ml, preferably in an amount of 5 to 100 $\mu$g/ml and most preferably in an amount of 10 to 50 $\mu$g/ml.

In a preferred embodiment of the present invention the sensor is a quartz crystal microbalance with a gold coating and a linker coating on top of the gold coating that is a self-assembled monolayers comprising at least one monothiol and at least one omega-mercaptocarboxylic acid, wherein the detection element comprises at least one antibody and the cell medium comprises at least one signal amplifier.

Analyte

As stated above, the detection element comprises at least one compound that specifically binds to an analyte, wherein the analyte may be any substance that is an indicator for the presence of one or more microorganisms. The substance that is an indicator for the presence of the microorganisms may be anything that points directly to the presence of the microorganism. It may be the microorganism itself or any substance expressed by the microorganism or any substance known to be produced in the environment of the cell medium or the environment the sample is taken from, by the microorganism or by another microorganism as a result of the presence of the microorganism to be detected and/or quantitatively measured. The analyte may for example also be a metabolite of the microorganism.

In a preferred embodiment the detection and quantitative measurement of the microorganism is a direct detection and/or measurement of the microorganism with the detection element, i.e. the microorganism itself is bound to the detection element and thereby generates a signal that is detected by the measuring device. In this embodiment the microorganism may be bound directly to the detection element or it may be bound by means of an intermediate compound that can bind to the microorganism and the detection element, i.e. an antibody, a part thereof or a modified antibody. In a more preferred embodiment, the detection and/or quantitative measurement of the microorganism comprises the direct binding of the microorganism to the detection element. This bond may be any bond, including a covalent bond, a dielectric bond or a van der Waals bond or multiple bonds thereof. This includes a bond between an antibody and an antigen thereof, which is most preferred.

In an alternative embodiment the detection and/or quantitative measurement of the microorganism is an indirect detection and/or indirect quantitative measurement of the microorganism. In this embodiment the microorganism is not bound to the detection element or the sensor in any way, neither directly nor indirectly. In this embodiment any other substance that is an indicator for the presence of the microorganisms is bound to the detection element. Therefore, in this embodiment the detection and/or quantitative measurement of the microorganism does not comprise the binding of the microorganism with the detection element. It does also not comprise the binding of the microorganism with any other part of the sensor. In a preferred embodiment of the present invention, the detection and/or quantitative measurement of the microorganism comprises the binding of compounds with the sensor that are produced (and expressed) by the microorganism or produced because of the presence of the microorganism. In this embodiment the microorganism may not be present in the cell medium. The microorganism may also not be present in the sample. The microorganism may also have already died off completely before taking a sample. The microorganism may therefore not be present in either the biological sample or the cell medium. In this case the previous presence of the microorganism can still be detected in the biological sample if the analyte is not the microorganism, but a substance that is an indicator for the presence of the microorganisms, e.g. a substance expressed by the microorganism or a substance known to be produced in the environment the biological sample is taken from or even the corpus of the dead microorganism or a part thereof or a metabolite. In this regard the present invention may also be used to detect and/or measure microorganisms that have artificially been altered with DNA or RNA, e.g. vectors and the like and that express specific molecules, e.g. proteins that may be detected by the sensor.

The microorganisms to be detected in the measurement device of the present invention or the method of the present invention (discussed below) may be from any field of technology or industry where complex biological sample need to be accessed and microorganism need to be measured. The present invention may, for example, be used in biology, medicine, pharmacy, food industry, including production and restaurants, environmental field and any other scientific, technical or industrial fields that requires the measurement of microorganisms and may be used to detect microorganisms in samples from all these fields.

In one embodiment, the analyte is selected from the group of pathogenic microorganisms, parts thereof, non-pathogenic microorganisms, parts thereof, compounds expressed from pathogenic microorganisms or non-pathogenic microorganisms and antibody complexes comprising antibodies and pathogenic microorganisms or non-pathogenic microorganisms. In a preferred embodiment, the analyte is selected from the group of pathogenic microorganisms, parts thereof, non-pathogenic microorganisms, parts thereof, compounds expressed from pathogenic microorganisms or non-pathogenic microorganisms and antibody complexes comprising antibodies and pathogenic microorganisms or non-pathogenic microorganisms. Preferably the analyte is selected from the group consisting of pathogenic microorganisms and non-pathogenic microorganisms and more preferably the analyte is selected from the group consisting of pathogenic microorganisms. In an even more preferred embodiment the analyte is a bacterium. Most preferably it is selected from the group consisting of the genera *Pseudomonas* and *Listeria* and the species *Escherichia coli, Salmonella Typhimurium, Bacillus cereus* and *Francisella tularensis* and preferably the species *Listeria monocytogenes.*

The analyte(s) may be a single microorganism, i.e. in a specific detection or even a single strain or it may be a plurality of microorganisms. e.g. all or several strains of a species or all or several species of a genus of microorganisms. The analytes may have common properties that allow detection by the same detection element, as for example all species of a genus or a family and the like or any other property that they share and that allow binding by the same detection element. They may also have no common properties and it may not be able to bind them to the same detection element. In this case a plurality of detection elements may be used on the same or on a plurality of sensors to detect a plurality of analytes.

The analyte of the present invention may be a microorganism that is not viable, i.e. dead. The same is true for microorganisms that are viable, but not culturable (VBNCs). In these cases, the concentration in the cell medium must be at or above the LOD of the sensor.

In a preferred embodiment of the present invention the sensor is a quartz crystal microbalance with a gold coating and a linker coating on top of the gold coating that is a self-assembled monolayers comprising at least one monothiol and at least one omega-mercaptocarboxylic acid, wherein the detection element comprises at least one antibody, the cell medium comprises at least one signal amplifier and the one or more analytes comprises or are at least one microorganism. In a preferred embodiment of the present invention the sensor is a quartz crystal microbalance with a gold coating and a linker coating on top of the gold coating that is a self-assembled monolayers comprising at least one monothiol and at least one omega-mercaptocarboxylic acid, wherein the detection element comprises at least one antibody, the cell medium comprises at least one signal amplifier and the one or more analytes comprises or are at least one bacterium.

Cell medium

As stated above, the cell medium of the measuring device of the present invention is a culture medium that supports the growth of the microorganisms to be detected or quantitatively measured or both. All cell media and ingredients for cell media known in the art for the microorganism to be detected may be used. Also, any kind of cell media may be used that supports the growth of the microorganism to be measured. The culture medium may for example be selected from the group consisting of nutrient media, selective media, non-selective media, minimal media, and differential media and preferably is selected from the group consisting of nutrient media, selective media and non-selective media and more preferably it is a selective nutrient medium. A general cell medium may be used that supports the growth of a wide variety of microorganisms. As preferred example tryptic soy broth may be mentioned. In a preferred embodiment of the present invention however, the culture medium is a selective medium that selectively supports the growth of the one or more microorganisms to be detected and/or quantitatively measured. This can be done by any known method, for example by addition of toxic substances, e.g. antibiotics, against which the microorganism to be measured is resistant. This embodiment has various advantages. It improves accuracy of the measurement by fostering growth of the microorganism to be detected and/or quantitatively measured, but not of other microorganisms. Other microorganisms may interact with the sensor or with the microorganism to be detected and/or quantitatively measured. They may interact with the sensor by forming a precipitation on its surface which would result in the erroneous or inhibited detection of the presence of the microorganism to be detected and/or quantitatively measured. They may also alter the surface of the sensors in other ways. In addition, the other microorganism may secrete substances that influence the growth of the microorganism to be detected and/or quantitatively measured, they may especially express harmful substances that inhibit its growth and may therefore lead to erroneous measurement or may even make measurement impossible. The cell medium may also comprise compounds that impede growth of microorganisms other than the one or more analytes.

In a more preferred embodiment of the present invention the sensor is a quartz crystal microbalance with a gold coating and a linker coating on top of the gold coating that is a self-assembled monolayers comprising at least one monothiol and at least one omega-mercaptocarboxylic acid, wherein the detection element comprises at least one antibody, the cell medium comprises at least one signal amplifier, the one or more analytes comprises or are at least one microorganism bacterium and the cell medium comprises tryptic soy broth. In a more preferred embodiment of the present invention the sensor is a quartz crystal microbalance with a gold coating and a linker coating on top of the gold coating that is a self-assembled monolayers comprising at least one monothiol and at least one omega-mercaptocarboxylic acid, wherein the detection element comprises at least one antibody, the cell medium comprises at least one signal amplifier, the one or more analytes comprises or are at least one bacterium and the cell medium comprises tryptic soy broth.

Temperature

As known in the art, the temperature may have a considerable influence on the signal of the sensor and the growth of microorganisms in the cell medium. The measuring device of the present invention therefore preferably comprises means for temperature control. More specifically the measuring device is especially preferred with means to regulate the temperature of the cell medium and the sensor. Any known device may be used to heat or cool the measuring device and/or the cell medium and /or the sensor and any known device may be used to regulate the temperature, i.e. one or more devices for measuring the temperature of the cell medium, one or more devices for heating the cell medium, one or more devices for cooling the cell medium and one or more controllers to control heating and cooling devices and so on. In a preferred embodiment the measuring device and/or the measuring cell is an incubator or is comprised in an incubator.

Temperature control may comprise keeping the temperature at a predetermined temperature (set temperature), i.e. 37 °C. It may also comprise application of a temperature profile to optimize microorganism growth. If a temperature profile is used, the measuring device of the present invention must be calibrated with the same microorganism to be measured, the same temperature profile and the same sensor. The temperature may be controlled in a range of +- 5 °C around a predetermined temperature, preferably in a range of +- 2°C and more preferably in a range of +- 1 °C. The best temperature may also depend on the microorganism to be detected and can easily be determined by preliminary tests.

Continuous measurement

A simple detection of the microorganisms may be performed as a non-continuous measurement by simply bringing the sensor into contact with the cell medium and determine the change of the signal produced by the sensor. This signal change may then be compared with a calibration curve which is measured for this measurement beforehand. However, for other measurements, for example in order to measure very small concentrations of microorganisms or to determine if the microorganisms are viable and culturable, a continuous measurement may be performed, if necessary. A continuous measurement is a preferred embodiment of the present invention. In such a measurement the cell medium which is a culture medium allows the microorganisms to proliferate and the change in the concentration of the one or more microorganisms is measured continuously. The one or more microorganisms to be selectively detected and/or selectively quantitatively measured are therefore cultured in the measuring cell. During culturing they are selectively detected and/or selectively quantitatively measured. Continuous measurement therefore also preferably comprises at least that the one or more microorganisms to be selectively detected or to be selectively quantitatively measured are continuously cultivated in the measuring cell. The microorganisms in the cell medium and in the measuring cell are not killed by the measurement or for the purpose of taking a measurement. Neither the cell medium nor the one or more microorganisms are influenced by the measurement. For a continuous measurement the measuring device of the present invention must comprise means for continuous detection. Therefore, in a preferred embodiment of the present invention, the measuring device comprises means for continuous measurement. Such equipment for continuous measurement requires at least that the cell medium is a culture medium that supports the growth of the microorganisms to be detected or quantitatively measured or both. Such equipment preferably also comprises one or more or all devices of the group consisting of one or more devices for measuring the temperature of the cell medium, one or more devices for heating the cell medium, one or more devices for cooling the cell medium, one or more devices for measuring the pH value of the cell medium, one or more devices for adding acids to the cell medium, one or more devices for adding bases to the cell medium, one or more devices for adding buffer to the cell medium, one or more stirrer and one or more controller.

Controller

In preferred embodiments, the measuring device of the present invention comprises one or more controller. The one or more controller read out the signal of the sensor. It may also read out, if present, the signals of one or more or all of the devices of the group consisting of one or more devices for measuring the temperature of the cell medium, one or more devices for heating the cell medium, one or more devices for cooling the cell medium, one or more devices for measuring the pH value of the cell medium, one or more devices for adding acids to the cell medium, one or more devices for adding bases to the cell medium, one or more device for adding buffer to the cell medium and one or more stirrer. It may also control these devices to control the temperature, the pH value and the stirring in a desired range.

Preparation of sensors

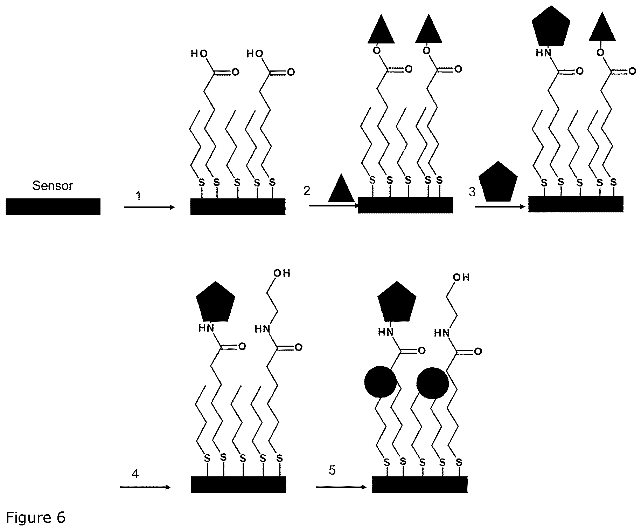

Any known sensor that allows selective detection of microorganisms may be used. The sensors used in the measuring device and the method of the present invention are in principle known. A preferred embodiment of the sensor of the present invention is illustrated in Figure 6. As can be seen in a first preparation step (step 1) the sensor (black rectangle) that may already have been coated with gold and may or may not have an adhesive layer between the coating and the sensor, is first cleaned, preferably with a mixture of concentrated sulfuric acid and 30% hydrogen peroxide typically for 3 to 30 minutes and reacted with two types of thiols, a monothiol and an omega-mercaptocarboxylic acid. Depending on the sensor surface and the linker, the sensor may be treated with solutions of the linker in suitable solvents, e.g. ethanol/ water 2:1 (vol%) or pure ethanol. The concentration of the linker in the solution is preferably in the range of 0.1 mM to 50 mM and the treatment time is preferably 1 to 24 h. Any monothiol and any omega-mercaptocarboxylic acid described herein may be used. Both thiols used usually have no further functional groups, but may in principle have further functional groups. The latter is not preferred. The thiols are part of the linker coating that allows to immobilize the detection element on the sensor. The monothiol and the omega-mercaptocarboxylic acid preferably have a straight hydrocarbon backbone and a carbon number in the range of 2 to 30, preferably 3 to 20, but as stated above, in principle any monothiol and any omega-mercaptocarboxylic acid may be used. The detection element is bound to the carboxylic acid groups and the monothiol with no further functional groups provides a linker coating on the sensor that prevents close contact of the cell medium and especially of the microorganisms comprised therein with the sensor material or the coating thereof.

The carboxyl groups are then activated by reagents (black triangle) known in the art (step 2). In a preferred embodiment of the present invention carbodiimides may be used to activate the carboxyl groups. The carbodiimides may be selected from the group consisting of 1-Ethyl-3-(3-dimethylaminopropyl)carbodiimide (EDC), 1-Ethyl-3-(3-dimethyl-laminopropyl)carbodiimide/ (sulfo) N-Hydroxysuccinimid, N,N'-Dicyclohexylcarbodiimid (DCC) and *N,N'*-diisopropylcarbodiimide (DIC). EDC is preferred.

In a subsequent step coupling of a detection element (black pentagon) to the linker takes place (step 3). Preferably the detection element is an antibody or a binding protein that is able to bind to a compound comprising a binding site, especially an antibody, as described above. A preferred example of such a binding protein is protein A. In this step the activated carboxyl group reacts with a primary or secondary amino group of the antibody or the protein that is able to bind to a compound comprising a binding site to form an amide. This chemistry is well known in the art. The antibody provides the method of the present invention with the selectivity it needs. Depending on the active group and the protein, 30 minutes to 20 hours coupling (reaction) time with a 5 to 100 µg/ml aqueous solution of the detection element may be appropriate. Lower concentrations and lower treatment times may lead to weaker signals. Remaining activated groups are deactivated by quenching (step 4) and in a final step blocking takes place (step 5). These two steps ensure that all possible binding sites on the surface of the linker coating apart from the detection element are inactivated. Quenching inactivates all remaining activated carboxyl groups for example and preferably by bringing them into contact with a reactive amine like ethanolamine thereby forming amide groups. Depending on the active group and the compound used for quenching, a treatment with 10 mM to 10 M solutions of the reagent for 10 minutes to 3 hours may be appropriate. Blocking entails inactivation of all sites that may form non-covalent bonds on the surface of the linker to ingredients of the cell medium. For this purpose, a protein may be added that forms a bond with such sites. Preferred substances that may be used as blocking agent may be selected from the group consisting of bovine serum albumin (BSA), casein and milk powder. Depending on the proteins used for quenching, the linker and the buffer, a treatment with a 0.1 to 5 wt-% solution of the blocking agent for 1 to 20 hours may be appropriate.

In a further preferred embodiment a linker with a terminal amino group is used, for example a linker as described herein, preferably cysteamine. Coupling of antibodies or a binding protein with such a linker may take place by use of glutaraldehyde, which likely form imides with the terminal amino group of the linker and a primary amino group of the antibody or a binding protein.

In another embodiment of the present invention, instead of antibodies binding proteins are coupled to the end groups of the linker, as described above. Subsequently quenching and blocking takes place as described above. These proteins are then able to bind to antibodies which serve as detection element. The antibodies are applied to the surface of the sensor after blocking in a separate reaction step. They may alternatively be introduced together with the cell medium. The antibodies then serve as the compound of the detection element that specifically binds to an analyte. This embodiment has already been described above and provides high flexibility to the present method as it allows to easily customize the sensor for each measurement.

Measuring cell

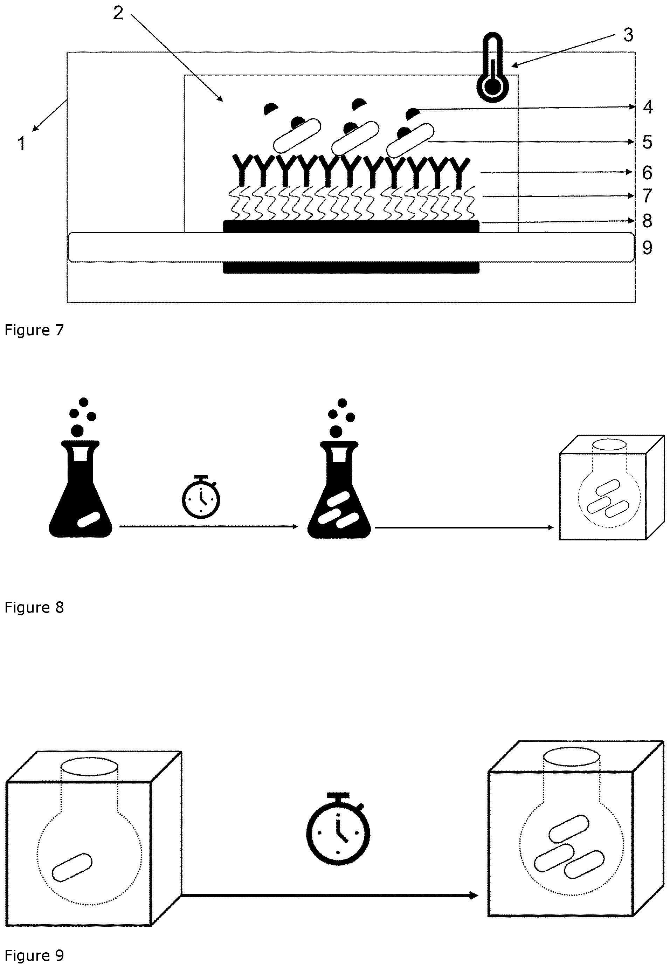

In the following we will describe an example of a measuring cell of the present invention and a method of the present invention for illustrative purposes. The measuring cell is shown in Figure 7. The outer square shows the outer casing of the measuring cell (1). The outer dimensions of the measuring cell used in the experiments of the present application was about 40x40x40 mm. A measuring cell useful in the present invention can usually accommodate a volume of cell medium in the range of 10 μL to 10 mL. In the casing, a sensor (6, 7, 8, 9) is integrated, consisting of a blank sensor (9), i.e. the sensor material that is provided with a coating (8) and possibly with an adhesive layer between the coating and the sensor (not shown). The sensor is preferably a QCM sensor. The schematic representation shows only one possible configuration of the measuring cell, where the sensor is placed horizontally at the bottom of the cell. The sensor can be placed at any part of the measuring cell in any orientation, horizontally top or bottom or vertically whereas the part of the sensor with the detection element is in contact with the cell medium. Installing the sensor such that the part of the sensor where the detection element is located does not point to the top of the measuring cell may have the advantage that depositions of solids from the cell medium are not deposited on top of the detection element. The sensor is also provided with a linker (7) coating and a detection element (6), which may be an antibody. The measuring cell (1) is also provided with a reservoir in which the cell medium (2) is comprised. As can be seen the cell medium (2) comprises the analyte (or target, 5) and a signal amplifier (4). The detection element (6) selectively binds the analyte (5). The signal amplifier (4) is bound to the analyte (5) and part thereof is still unbound in the cell medium (2). In case the sensor is a QCM sensor, the signal amplifier (4) provides the analyte (5) with more mass in order to amplify the sensor signal. In other embodiments the signal amplifier (4) may also provide the analyte with fluorescence properties or any other properties that may be useful for the detection of the analyte. In addition, the measuring cell (1) comprises means for temperature control, which is symbolized by the thermometer (3). In this embodiment a sensor changes its frequency depending on the amount of analyte bound to the sensor, which depends on the amount of analyte present in the cell medium and the amount of signal amplifier bound to the analyte.

Method

A further aspect of the present invention is a method for the detection of one or more microorganisms comprising the following steps:

- providing a measuring device that comprises a measuring cell that comprises one or more sensors, wherein

  - one or more detection elements are immobilized on at least one part of a surface of each of the one or more sensors and

  - the one or more detection elements comprise at least one compound that selectively binds to at least one of one or more analytes, wherein the one or more analytes may be any substance that is an indicator for the presence of the one or more microorganisms and the one or more sensors are able to detect the binding of the at least one of the one or more analytes to the sensor,

- introducing a cell medium into the measuring cell,

- at least one part of a surface of each of the one or more sensors on which the one or more detection elements are immobilized is brought into contact with the cell medium, and

- introducing one or more analytes into the cell,
  characterized in that

- the cell medium is a culture medium that supports the growth of the one or more microorganisms to be detected

- the pH value of the cell medium is controlled throughout the measurement.

The method of the present invention has the same advantages as described above for the measuring device of the present invention. All the elements used in the method of the present invention are the same as the elements of the measuring device as stated above, including measuring cell, sensor, detection element and cell medium. Their definitions and embodiments also apply to the method of the present invention. In a preferred embodiment the measuring device of the present invention is used in the method of the present invention.

The wording that the pH value of the cell medium is controlled includes the method where a buffer is added to the cell medium, which keeps the pH value in a certain range. It also includes adding acids, bases and/or buffer as necessary to adjust the pH to the desired value. In one embodiment the pH value of the cell medium is monitored, for example by one or more pH meters and acids, bases and/or buffer as necessary to adjust the pH to the desired value are added to the cell medium. In a preferred embodiment the pH value is controlled throughout the whole measurement.

Continuous measurement

The method of the present invention may be performed as a non-continuous measurement, where the sensor is brought in contact with the sample and the sensor produces a signal equivalent to the amount of the analyte as described above for the measuring device of the present invention. Alternatively, the method may be performed as a continuous measurement as described above for the measuring device of the present invention. This is one of the main advantages of the present invention.

Continuous measurements are known in the art for example the measurement of the optical density. The known methods are not selective or specific for a certain microorganism of a group thereof. Measurement methods that are selective and/or specific require direct contact of the sensor with the medium the microorganisms are comprised in, as discussed above. This is also true for the method of the present invention. The prior art methods suffer from the problem that non-specific depositions on the sensor occur that derive from complex biological ingredients in the measuring cell. The present method inhibits non-specific depositions on the sensor. In contrast to the prior art in the present invention the pH value is controlled during the continuous measurement of the sample while the sample is incubated. Surprisingly, this leads to the inhibition of non-specific depositions on the sensor.

It follows that in contrast to the prior art, the present method can be operated continuously, without non-specific deposition on the sensor, which makes it possible to detect microorganisms at any time during enrichment, i.e. continuously. Usually, in the art, enrichment steps are necessary to increase the number of bacteria to a level that is above the detection limits of the method of choice. If the microorganism concentration is high in the initial sample, a detection could be possible earlier during enrichment, as the bacteria would reach numbers above the detection limits of the sensor earlier. However, in an endpoint method, i.e. a non-continuous method, an earlier detection is not possible, as the protocol foresees an incubation for the predefined time. This is necessary to safely obtain a positive result even if the starting concentration of the microorganisms is very small. That is different in continuous measurements, where the concentration may be monitored at any time throughout the experiment and the microorganism can be detected immediately when the detection limits for the microorganisms are exceeded. This method delivers the information of a contamination at the earliest possible time. The inventive method allows manufacturers to intervene earlier than in endpoint measurements. Moreover, continuous measurements record changing signals that are correlated to the proliferation of a microorganism and can confirm its viability directly. Non-continuous measurements, i.e. endpoint measurement, are taken at the end of the incubation time and may measure dead microorganisms only. Only a signal obtained in a continuous measurement or a comparison of results at different points in time in a favorable environment for bacterial growth is proof that the bacteria proliferate.

Also, in contrast to endpoint measurements, for continuous measurements that detect microorganisms directly during growth, no further preparation steps and chemicals are necessary for the measurement itself. Therefore, continuous measurement methods that detect microorganisms during growth, exhibit a simpler workflow and are faster than endpoint measurements. State-of the-art measurement methods are not capable of selectively detecting a target microorganism in a complex growth medium directly, which is circumvented so far by using endpoint measurements, which entails, handling of the sample for enrichment, possibly multiple times, subsequent handling of the enriched sample for measurement and usually also diluting the enriched matrix for measurement and thereby lowering the LOD of the measurement. The advantages of continuous measurements are therefore obvious.

It is another advantage of the method of the present invention that it allows to determine if the microorganism is alive, qualitatively and/or quantitatively. Since the cell medium is a culture medium, if microorganisms that are able to proliferate are present, the microorganisms will grow in the culture. The signal output of the sensor will therefore change over time with the growth of the microorganism.

In this embodiment, in some cases, especially for large starting concentrations of the analyte it is not only possible to determine if the microorganisms are alive, it is also possible, to quantitatively measure the concentration of the microorganisms alive and the concentration of the microorganisms that are not. The signal of the sensor at the beginning of the measurement is a measure for the overall concentration of the microorganism in the cell medium and the change of the sensor signal over time is a measure for the concentration of the microorganisms that are alive. Calibration of the sensor is possible by use of cell medium with a known amount of dead microorganisms and/or microorganisms that are alive and can proliferate.

The signal of the sensor is temperature dependent. In addition, the growth of the microorganisms is temperature dependent. For this reason, in a preferred embodiment of the method of the present invention the temperature is controlled. Preferably the temperature is kept at a fixed predetermined temperature or in a tight range around such a fixed predetermined temperature. The temperature is best kept in a range of no more than +-3 °C of the predetermined temperature and more preferably in a range of no more than +-2 °C and most preferably in a range of no more than +-1 °C of the predetermined. In other embodiments the temperature may be changed during the measurement, for example to influence the growth of the microorganisms or to influence the expression of substances that may be used as analyte. In such a case calibration may be done with the same temperature profile as used during the measurement. The predetermined temperature is preferably in the range of 20 to 45 °C and more preferably in the range of 25 to 40, even more preferably in the range of 30 to 39 °C. Most preferably the predetermined temperature is 37 °C. This is the ideal temperature for growth of most microorganisms.

In a more preferred embodiment, the one or more analytes comprise at least one microorganism bacterium, the measurement is continuous and the temperature is controlled at a predetermined temperature the range of 25 to 40 °C. In a more preferred embodiment, the one or more analytes comprise at least one bacterium, the measurement is continuous and the temperature is controlled at a predetermined temperature the range of 25 to 40 °C.

In a preferred embodiment of the present invention the sensor is a quartz crystal microbalance with a gold coating and a linker coating on top of the gold coating that is a self-assembled monolayers comprising at least one monothiol and at least one omega-mercaptocarboxylic acid, wherein the detection element comprises at least one antibody and the measurement is continuous.

In a preferred embodiment of the present invention the sensor is a quartz crystal microbalance with a gold coating and a linker coating on top of the gold coating that is a self-assembled monolayers comprising at least one monothiol and at least one omega-mercaptocarboxylic acid, wherein the detection element comprises at least one antibody, the measurement is continuous and temperature is controlled at a predetermined temperature the range of 25 to 40.

In a further preferred embodiment of the present invention the sensor is a quartz crystal microbalance with a gold coating and a linker coating on top of the gold coating that is a self-assembled monolayers comprising at least one monothiol and at least one omega-mercaptocarboxylic acid, wherein the detection element comprises at least one antibody, the measurement is continuous and the one or more analytes comprises or are at least one microorganism. In a further preferred embodiment of the present invention the sensor is a quartz crystal microbalance with a gold coating and a linker coating on top of the gold coating that is a self-assembled monolayers comprising at least one monothiol and at least one omega-mercaptocarboxylic acid, wherein the detection element comprises at least one antibody, the measurement is continuous and the one or more analytes comprises or are at least one bacterium.

In a more preferred embodiment of the present invention the sensor is a quartz crystal microbalance with a gold coating and a linker coating on top of the gold coating that is a self-assembled monolayers comprising at least one monothiol and at least one omega-mercaptocarboxylic acid, wherein the detection element comprises at least one antibody, the cell medium comprises at least one signal amplifier, the one or more analytes comprises or are at least one bacterium.

Use

A further aspect of the present invention is the use of the measuring device and/or the method of the present invention for the measurement of microorganisms in a sample from the field of biology, medicine, pharmacy, food industry, including production and restaurants, environmental field, pathology and any other scientific, technical or industrial fields that requires the measurement of microorganisms. To treat infectious diseases appropriately (e.g. by antibiotics) it is of paramount importance to identify the pathogen. The pathogens may be present in a patient's body, where the patient may be human or an animal. It is also important to identify pathogens in environments where medical practitioners treat diseases, e.g. in a hospital environment and in medical practices. An especially important field for the application of the present invention is the detection of hospital germs, especially when they are resistant against antibiotics.

A preferred embodiment of the present invention is the use of the measuring device and/or the method of the present invention in the detection of pathogens. A further preferred embodiment of the present invention is the use of the measuring device and/or the method of the present invention in the quantitative measurement of pathogens. In a further preferred embodiment, the measuring device and/or the method of the present invention are used in the diagnosis of diseases, preferably diseases derived from hospital germs. In a very much preferred embodiment, the analyte of the present invention is comprised in a sample from a human body, i.e. human tissue or human body fluid or from a hospital or a medical practice. In a very much preferred embodiment, the method of the present invention comprises the step of adding a sample to the cell medium, wherein the sample is a sample from the body of a mammal, preferably a human body. In a very much preferred embodiment, the analyte of the present invention is comprised in a food sample or an environmental sample, i.e. swab sample from the environment in or close to a critical infrastructure such as a food production site or hospital. In a very much preferred embodiment, the analyte of the present invention is comprised in a food, e.g. a swab sample from the environment in or close to a site where food is processed. In a very much preferred embodiment, the method of the present invention comprises the step of adding a sample to the cell medium, wherein the sample is a sample from the environment in or close to a site where food is processed. In a very much preferred embodiment, the analyte of the present invention is comprised in an environmental sample, e.g. a swab sample from the environment in or close to a site where food is processed or where medical treatment is administered, e.g. hospitals, doctors offices or pharmacies. In a very much preferred embodiment, the method of the present invention comprises the step of adding a sample to the cell medium, wherein the sample is a sample from the environment in or close to a site where food is processed or where medical treatment is administered, e.g. hospitals, doctors offices or pharmacies.

Measurement strategies

The method of the present invention may be performed with a number of measurement strategies. In the following some preferred embodiments are illustrated. The symbols in the figures the following text refers to are the same as in figure 7.

Figure 8 shows a method of the present invention with external enrichment. The microorganisms (rod-shaped) are enriched externally in a growth medium for a time sufficient to enrich the analyte to allow measurement thereof. As discussed above the analyte(s) may be microorganisms or any substance that are an indicator for the presence of the microorganisms. The enrichment phase usually lasts for several hours, preferably for 1 to 24 hours. During and/or at the end of the enrichment, samples are taken and measured in the measurement device of the present invention with the method of the present invention. The enriched sample is mixed with a buffer and given into the measuring cell (right side, cuboid). The mixing may take place in the measuring cell, in which case the buffer is preferably placed in the measuring cell and the enriched sample is added. It is important to keep the pH value stable at the time of mixing, especially when the mixing takes place in the measuring cell. Otherwise, non-specific deposits on the sensor may occur and the measurement is then no longer selective! Here, the concentration of the microorganism is determined at a specific point in time, after growth has occurred, if necessary. The sample has no contact with the sensor during growth. The sample is not measured continuously during growth, but at selected points in time. However, growth of the microorganisms and therefore also the fraction of the microorganisms that are alive may be measured by measuring at a plurality of points in time. This configuration is advantageous as it can deliver fast responses without sample preparation. Moreover, in cases when the culture medium is incompatible with the biosensor, it can be diluted before introducing it into the measuring cell to a concentration that allows measurement. The sample may also be measured continuously after insertion into the measuring cell. In this case incubation without contact to the sensor may take place for a time interval, in which a signal is likely not to be expected. This method limits the contact time of the sensor with the sample and therefore minimizes the time during which the sensor may be disturbed, altered and/or polluted by the cell medium. This method may extend the service life of the sensor.

Figure 9 shows a method of the present invention with internal enrichment in the measuring cell (cuboid). In this embodiment the microorganisms (rod-shaped) are enriched in the measuring cell. The cell medium is in permanent direct contact with the sensor during measurement. The cell medium is an optimized culture medium (nutrient medium) in which the microorganisms grow well. The pH value is kept stable during growth, preferably by buffering. In this embodiment growth of the microorganism can be measured continuously, which also provides evidence of living and culturable microorganisms. The pH value of the cell medium is important. The pH value is preferably kept stable during measurement!

In principle, a measurement with internal or external enrichment may be performed with or without a signal amplifier.

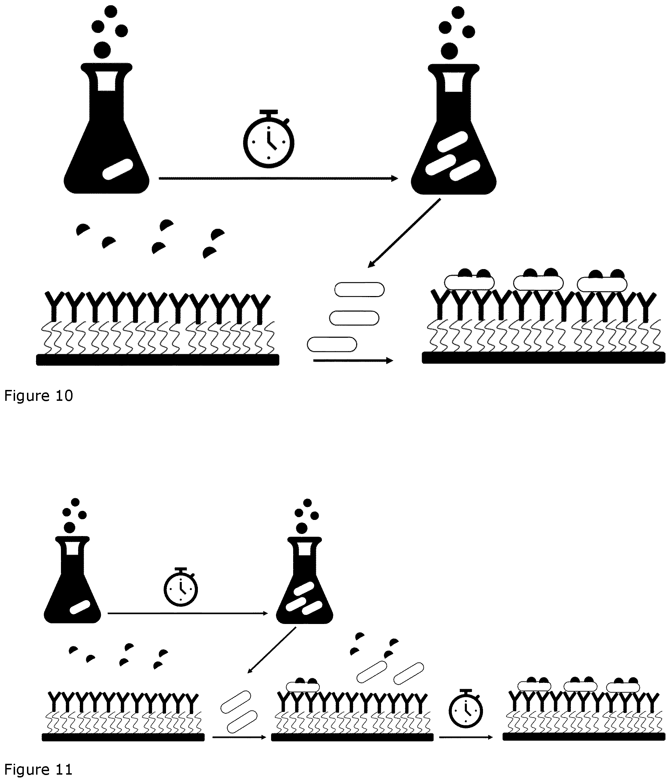

Figure 10 shows an embodiment of the method of the present invention with external enrichment outside the measuring cell (not shown) prior to measurement and the use of a signal amplifier (semicircular shape). The figure shows the sensor (black bar) with a linker (curved line) and a detection element (Y-shaped). The microorganisms are shown as rod-shaped elements.

In this embodiment after the preliminary enrichment of the microorganism outside the measuring cell, it is not necessary that any further growth takes place in the measuring cell. A signal amplifier (e.g. antibodies) selective for the microorganisms to be measured is placed in the measuring solution, the pH value of which is kept stable during and after addition of the sample to be measured. First a sample, such as swipe samples, food samples, environmental samples, medical samples, etc. is taken and external enrichment of this sample in a suitable medium is performed for a certain amount of time. In a second step all of the medium or an aliquot thereof enriched with the microorganism is added to the cell medium in the measuring cell that comprises a signal amplifier. The analyte (e.g. microorganisms) comprised in the aliquot binds to the detection element on the sensor and the signal amplifier in the cell medium. If the concentration of the analyte in the aliquot is high enough, a signal is recorded within minutes to a few hours after addition of the aliquot. Only a single sample transfer from the culture medium (nutrient medium) or its supernatant, if there is any deposition of matter in the culture, to the measuring cell is necessary. No further preparations, dilution, handling steps or the like are required. This simplifies the process and is an advantage over the prior art, especially in commercial settings.

Figure 11 shows an embodiment of the method of the present invention with external enrichment outside the measuring cell, the use of a signal amplifier and subsequent growth in the measuring cell. The symbols are as described in the previous paragraph for Figure 10. The measurement is performed as described in the previous paragraph for Figure 10. If the concentration of the microorganism is low, the microorganisms can be further enriched in the measuring cell (e.g. at 37°C) for a certain amount of time and the analyte and therefore growth of the microorganisms can be continuously measured during enrichment in the measuring cell. Detection of growth here means detection of living and culturable microorganisms. Only a single sample transfer from the nutrient medium to the measuring cell is necessary. No further preparation, dilution, handling steps or similar are required. The life time of the sensor may be increased (see also discussion above in regard to Figure 8) by this procedure as compared with a procedure where the whole enrichment takes place in the measuring cell as described in the following.

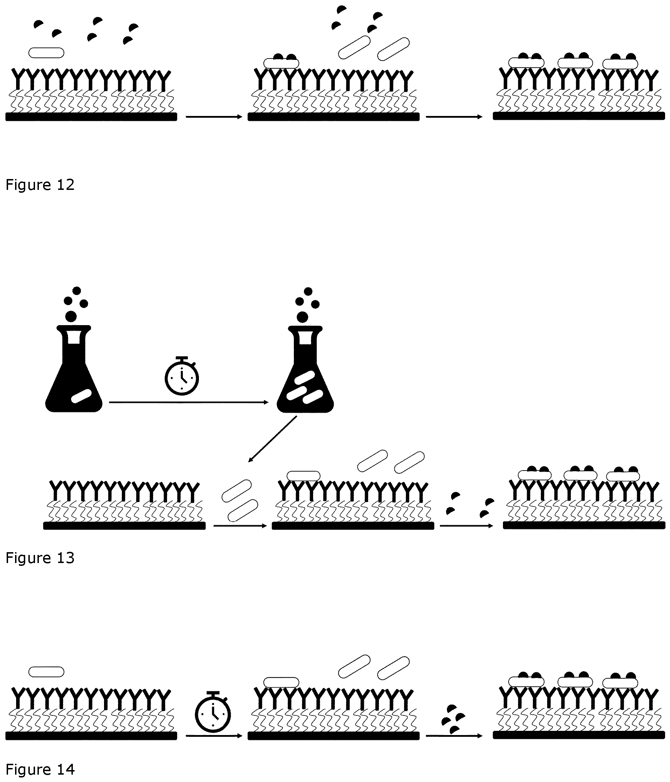

Figure 12 shows an embodiment of the method of the present invention without external enrichment outside the measuring cell, the use of a signal amplifier and microorganism growth in the measuring cell. The symbols are as described above for Figure 10. This method is performed as the foregoing measurement method shown in Figure 11, but without external enrichment. The sample is directly introduced into the cell medium and the microorganisms are enriched directly in the cell medium in a specific culture medium that keeps the pH value stable and comprises a signal amplifier (e.g. antibodies). The sample (environmental, surrounding, food sample or similar) can be measured in this nutrient medium solution in order to selectively and continuously track the growth of microorganisms if necessary (e.g. *Listeria*). Detection of growth means detection of living microorganisms. This is the simplest measuring method with the least amounts of handling steps and therefore highly preferably in industrial settings.

Figure 13 shows the implementation of a two-step assay for the method of the present invention. This method is very similar to the methods depicted in Figures 10 and 11, with the distinction that the signal amplifier is added to the cell medium after growth of the microorganisms. The symbols are as described above for Figure 10. Preferably but not necessarily the signal amplifier is added after removal of the cell medium, washing of the measuring cell, for example with buffer and introducing new buffer as cell medium. In this embodiment the signal amplifier does not come into contact with the sample or the complex cell medium used during growth. The microorganisms are externally enriched and an aliquot is transferred to the measuring cell. After incubation, i.e. binding of the analyte to the detection element, the signal amplifier is added to the cell medium or the cell medium is removed, the cell is washed and subsequently a solution of the signal amplifier is introduced into the cell medium. In a variation, the microorganisms may additionally be grown in the measuring cell after external pre-enrichment and before the signal amplifier is added or the measuring cell is washed and the signal amplifier is introduced. Introduction of the signal amplifier after removal of the medium in which the microorganism has been enriched avoids detrimental effects of the medium on the signal amplifier, e.g. aggregation on proteins or other ingredients of the medium, decomposition by extracellular enzymes and so on. In other words this procedure allows the use of signal amplifiers that may be lost during enrichment, because they interact with the environment of the cell medium and/or the growth medium. This procedure is especially useful for signal amplifiers that are unstable in the medium and/or that are very expensive.

Figure 14 shows another implementation of a two-step assay for the method of the present invention as described above for Figure 13. The symbols are as described above for Figure 10. In this embodiment no external enrichment takes place. Instead, the microorganisms are enriched in the measuring cell (usually over several hours). During enrichment the analyte binds to the detection element, then the signal amplifier is added or the cell medium is removed, the measuring cell is washed and a solution of the signal amplifier is introduced into the measuring cell after which the signal of the sensor is recorded.

**Brief description of the figures**

Figure 1: Figure 1 shows the measurement of the growth of *Listeria monocytogenes,* over time with a sensor of the present invention that is specific for the species *Listeria monocytogenes,* together with the measurement of other bacteria for which the sensor is not specific.

Figure 2: Figure 2 shows the bacterial growth of *Listeria monocytogenes* with different starting concentrations of the bacteria, measured with a sensor of the present invention that is specific for the species *Listeria monocytogenes.*

Figure 3: Figure 3 shows the bacterial growth for various species of the genus *Listeria,* measured with a sensor of the present invention that is selective for the genus *Listeria.*

Figure 4:      Figure 4 shows the bacterial growth for various species which are not of the genus Listeria, measured with a sensor of the present invention that is selective for the genus *Listeria.*

Figure 5:      Figure 5 shows the measurement of the growth of *Escherichia coli,* over time with a sensor of the present invention that is selective for the species *Escherichia coli,* together with the measurement of other bacteria for which the sensor is not selective.

Figure 6:      This figure shows a known method to produce an example of a sensor of the present invention.

Figure 7:      Figure 7 shows an example of a measuring cell of the present invention.

Figure 8:      Figure 8 shows an embodiment of the method of the present invention with external enrichment.

Figure 9:      Figure 9 shows a preferred embodiment of the method of the present invention with internal enrichment in the measuring cell.

Figure 10:      Figure 10 shows an embodiment of the method of the present invention with external enrichment outside the measuring cell and the use of a signal amplifier.

Figure 11:      Figure 11 shows an embodiment of the method of the present invention with external enrichment outside the measuring cell, subsequent growth in the measuring cell and the use of a signal amplifier.

Figure 12:      Figure 12 shows an embodiment of the method of the present invention with only internal enrichment in the measuring cell and the use of a signal amplifier.

Figure 13:      Figure 13 shows an embodiment of the method of the present invention which is a two-step assay with external enrichment.

Figure 14:      Figure 14 shows an embodiment of the method of the present invention which is a two-step assay without external enrichment.

Figure 15:      Figure 15 shows the result of comparative example 1. The results of measurements are shown using sensors without detection element with and without buffer.

Figure 16:      This figure shows the effect of the buffered medium. Measurement is performed with a sensor selective for the detection of *Escherichia coli* in buffered and non-buffered medium that comprise L. lactis.

Figure 17:      Figure 17 shows a comparison between two measurements, one with living bacteria and one with dead bacteria.

Figure 18:      This figure shows the results of an embodiment of the present invention in which in a one-step procedure two swap samples are measured. Enrichment and detection are performed in the measuring cell in one step.

Figure 19:      Figure 19 shows the quantitative detection of *Escherichia* coli with a sensor of the measuring device and the method of the present invention. 3 different starting concentrations were measured.

**Experimental Part**

*Materials*

Microorganisms

| Name | Source |
|------|--------|
| Bacillus licheniformis | CCM2145 |
| Bacillus pumilus | CCM2144 |
| Lactobacillus lactis | CCM1877 |
| Lactobacillus rhamnosus | NCTC10302 |
| Atlantibacter subterranean | DSM16208 |
| Staphylococcus epidermis | NCTC11047 |
| Listeria grayi | DSM20601 |
| Listeria innocua | NCTC11288 |
| Listeria seeligeri | DSM20751 |
| Listeria monocytogenes | DSM20600 |
| Escherichia coli | DSM18039 |

CCM: Czech Collection of Microorganisms, Department of Experimental Biology, Faculty of Science, Masaryk University, Kamenice 5, building E25, 625 00 Brno, Czech Republic

DSM: DSMZ-German Collection of Microorganisms and Cell Cultures GmbH, Inhoffenstraße 7B, 38124 Braunschweig

NCTC: National Collection of Type Cultures, UK Health Security Agency, Porton Down, Salisbury, SP4 03G, UK

Antibodies

(1) L. mono (anti-Listeriolysin O Antibody): Diatheva Srl, Cartoceto, Italy - Cod: ANT0006 (https://www.diatheva.com/product/rabbit-anti-listeriolysin-o-200/)
(2) Listeria: Thermo Fisher Scientific Inc., Waltham, Massachusetts, USA - Catalog# PA1-7230 (https://www.thermofisher.com/antibody/product/Listeria-monocytogenes-Antibody-Polyclonal/PA1-7230)
(3) *E. coli*: Thermo Fisher Scientific Inc., Waltham, Massachusetts, USA - Catalog# PA1-7213 (https://www.thermofisher.com/antibody/product/E-coli-serotype-O-K-Antibody-Polyclonal/PA1-7213)

Buffers

1xPBS: 0.14 M NaCl, 0.0027 M KCl, phosphate buffer pH 7.4 0.010 M
10xPBS: 1.4 M NaCl, 0.027 M KCl, phosphate buffer 0.1 M, pH adjusted to 7.6 (with HCl)
MES: 0.1 M MES, 0.9% NaCl, pH 4.7

Growth medium

Tryptic soy broth (TSB Medium, Merck KGaA, Darmstadt, Germany - Cod: 22092 Millipore)

Proteins

Protein A (Pierce™ recombinant Protein A; Thermo Fisher Scientific Inc., Waltham, Massachusetts, USA - Catalog# 77673)

Bovin Serum Albumin (BSA, Lyophilised pH 7 (Fraktion V); Th. Geyer GmbH&Co. KG, Renningen, Germany)

Further remarks

In the context of the following methods description, water and the water in aqueous solutions refer to deionized water (Millipore, 18.2 MΩ cm). All preparation steps were made at 22°C.

*Sensor Preparation*

Commercially available QCMs with gold electrodes were used (RenLux Crystal Ltd, Shenzhen, China, CHI 7.995 MHz QCM sensor, Au coated with Ti adhesion layer). The gold electrodes on the QCMs were first cleaned with Piranha solution, that was pre-mixed and consisted of three parts by volume of concentrated sulfuric acid (96%, ACS reagent, 95.0-98.0%, Sigma-Aldrich Austria, Saint Louis, USA) and one part by volume of a 30 % by weight solution of hydrogen peroxide (stabilized for synthesis, Merck KGaA, Darmstadt, Germany), for 10 minutes. Subsequently the electrodes were washed with distilled water, followed by ethanol (p.a.) before air drying. The following steps refer only to the gold surface that comes into contact with the cell medium. The cleaned gold surface of the sensor was immersed for at least 3 hours in a solution of 2:1 ethanol:water vol% containing 25 mM 11-mercapto-1-undecanol (97%, Sigma-Aldrich, Saint Louis, MO, USA) and 5 mM 16-mercaptohexadcanoic acid (98%, Sigma-Aldrich, Saint Louis, MO, USA). The mixture formed a self-assembled monolayer (SAM) that served as linker on the gold electrode. After the incubation the QCM was washed three times with ethanol and 3 times with 2-(N-morpholino)ethanesulfonic acid (MES) buffer solution. After washing, the carboxylic acid function of the 16-mercaptohexadcanoic acid was activated by immersion in 1-Ethyl-3-(3-dimethylaminopropyl)carbodiimide solution (EDC, 200 mM in MES buffer adjusted to pH 4.7) for 20 minutes followed by washing 3 times with MES buffer and 2 times with phosphate buffered saline solution (1xPBS, pH 7.4; 10 mM phosphate buffer, 3 mM KCl, tablet, pH 7.4, Merck KGaA, Darmstadt, Germany). In the next step, a 0.1 mg/ml protein A solution (Thermo Fisher Scientific Inc., Waltham, Massachusetts, USA, in 1xPBS) was added to the activated linker for 2 hours. Protein A represents also the linker layer, as it is used to couple the detection element. In the next step, the surface was washed with MES buffer and 1xPBS and remaining EDC activated surface groups were quenched by a 0.1 M ethanolamine solution (≥98%, Sigma-Aldrich, Saint Louis, MO, USA, diluted in 1xPBS) for 60 minutes. Finally, the surface of the gold electrode is blocked by adding a solution of 1% bovine serum albumin, BSA (Bovine Serum Albumine, Lyophilised pH 7, fraction V, Th. Geyer GmbH&Co. KG, Renningen, Germany), and 0.05% Tween20 (viscous liquid, Sigma-Aldrich, Saint Louis, MO, USA) in 1xPBS for up to 20 hours. After washing with 1xPBS, sensors were ready to be mounted into measuring cells. The sensors were either used immediately or stored in 1xPBS for up to two days at 4 °C. The final functional sensor forms in situ when antibodies were added to the cell medium. Part of the antibodies bind to the protein A at the sensor surface via the Fc region of the antibodies, with the remaining (unbound) antibodies serving as signal amplifiers in the cell medium. Antibodies that were used in such experiments are disclosed above under "materials". In general, the cell medium was added to the measuring cell, immediately after preparation of the sensor followed by addition of an antibody to the cell medium in an amount that a concentration of 20 or 40 μg/mL antibody (if not stated otherwise in the following examples) in the cell medium results, if not otherwise stated or apparent from the context, in the following the concentration of the antibodies refers to its concentration in the cell medium. The amount of the antibody and the concentration of the solution depends on the antibody and the sensor. For the examples of the present invention the aforementioned amounts and concentrations are appropriate. However, it is also possible to mix antibody and cell medium before adding the mixture to the measuring cell.

In alternative preparations antibodies (10-20 μg/mL) may be directly coupled to the EDC activated group of the linkers without the use of protein A.

*Measurement*

For measurement of bacterial growth, the sensors were mounted in a measuring cell which has a volume for the cell medium of about 900 μL. The measuring cell size (outer dimension) was 40x40x40 mm. The sensor was mounted vertically in the measuring cell. The buffered growth medium was prepared by mixing TSB (Tryptic soy broth, Merck KGaA, Darmstadt, Germany) with 10xPBS (Sigma-Aldrich, Saint Louis, MO, USA, pH adjusted to 7.6) in the ratio of 1:1, which was used as the cell medium. The buffered growth medium and the antibody (20 or 40 μg/mL, diluted in growth medium) as stated above under materials were added to the measuring cell and the sensor was connected to a controller. The whole system was equilibrated at 37°C in a pre-heated incubator (Model BD 115, Binder GmbH, Tuttlingen, Germany) until a stable sensor baseline was reached. After baseline stabilization the sample was added to the cell medium. Bacteria samples with a quantified concentration were added directly to the cell medium (except for swab samples). Typically, 50 μL bacteria sample was added to 450 μl of cell medium to give 500 μL final volume of the growth medium. Different concentrations of bacteria were obtained by diluting the stock solution with growth medium (if not state otherwise). Swabs were spiked with 70 μL of bacteria solution with known concentration of $10^2$ CFU/mL. After sample addition, the bacterial growth was continuously measured over time. The antibody in this experiment serves as part of the detection element and as signal enhancer, as the protein A on the surface of the sensor binds the Fc region of the antibody. The antibodies bound to protein A also bind to the microorganism to be detected (its antigen) via its antigen binding site and remaining free antibodies in solution, that are not bound by protein A, bind to the microorganism as well.

*Bacteria culturing*

Bacterial colonies were picked from plated agar plates (containing TSB Medium), transferred to glass cuvettes containing the TSB medium and cultivated overnight at 37°C in an incubator except for *Lactobacillus rhamnosus and Lactobacillus lactis,* which were cultivated at 30°C. The bacteria concentration was either determined by diluting in 1xPBS and plating on Agar plates (plates were prepared by autoclaving tryptic soy agar medium at 120 °C and 3 bar for 20 minutes and adding it to a Petri dish in a laminar flow cabinet) or determined by measuring the optical density at 600 nm (in a Hitachi U-2900 spectrophotometer versus growth medium). All steps were performed in a laminar flow cabinet (except OD measurements).

Example 1: *Listeria monocytogenes* Sensor

In this example a sensor was prepared according to the aforementioned procedure with protein A coupled to the linker. As detection element and signal enhancer 20 $\mu$g/mL of an antibody which has Listeriolysin O (indicator for the presence of Listeria monocytogenes, hemolysin) as antigen was used, i.e. antibody (1) as stated above under "materials". The measurement was performed as described above. In this procedure the same antibody was used as signal enhancer. Either *Listeria monocytogenes, Listeria innocua, Atlantibacter subterranea* or *Bacillus pumilus* was added (50 $\mu$L of $10^6$ CFU/mL to 450 $\mu$L of cell medium to a final concentration of $10^5$ CFU/mL) to the measuring cell, whereas the sensor exhibited already a stable baseline. Growth was continuously monitored.

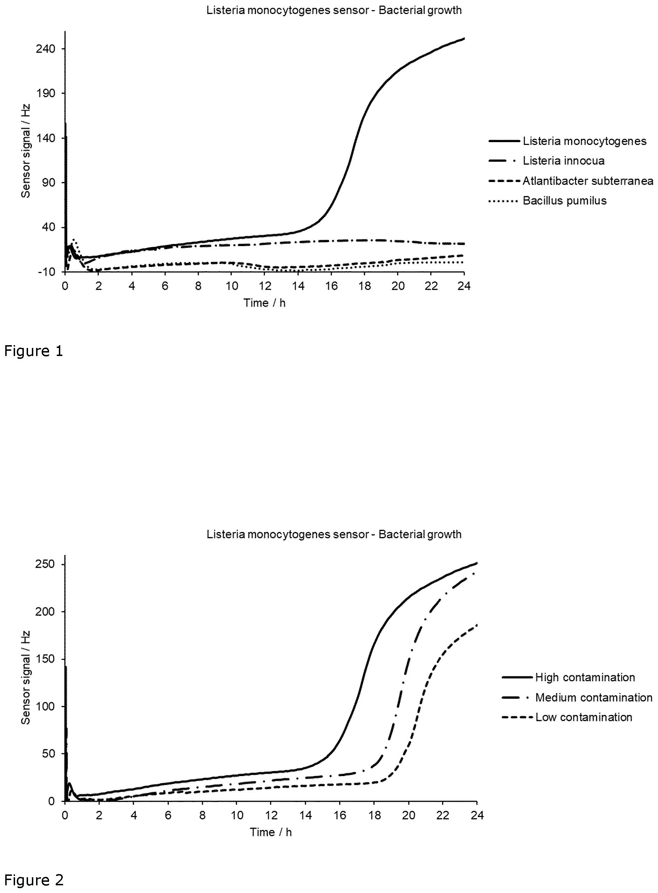

Figure 1 shows the results of experimental setups with anti-*Listeriolysin O* antibodies as detection elements and signal amplifiers. *Listeria monocytogenes* was detected in the cell medium, which is shown by the increase in the sensor signal after about 16 hours of incubation. Incubations with the same starting concentrations of *Listeria innocua, Atlantibacter subterranea* and *Bacillus pumilus* did not lead to an increased sensor signal, even after 24 hours of measurement. The sensor is therefore selective to *Listeria monocytogenes.*

In a further experiment the same procedure was repeated applying different concentrations of *Listeria monocytogenes.* As can be seen from Figure 2, different starting concentrations of *Listeria monocytogenes* led to increases of sensor signals at different times. The concentrations were $10^5$ CFU/mL in the cell medium for the example for high contamination, $10^3$ CFU/mL for medium contamination and $10^2$ CFU/mL for low contamination and were estimated by OD measurements. Higher concentrations were detected earlier than lower ones. The present apparatus and method can therefore be used to determine the bacteria concentration. It can also be seen that the time difference between the detection of the low contamination sample and the high contamination sample is more than 4 and a half hours. It is therefore possible to detect high contamination samples earlier than low contamination samples. The present invention is therefore superior to methods of the prior art that are not continuous measurements and therefore have to wait for a fixed time period before measurements can commence. The fixed amount of time has to be chosen such that even starting from the lowest concentrations (i.e. 1 CFU) a reliable result is obtained, i.e. the method of the prior art requires in any case the same time for enrichment (i.e. >22 h), no matter the starting concentration of the microorganism.

Example 2: *Listeria Sensor*

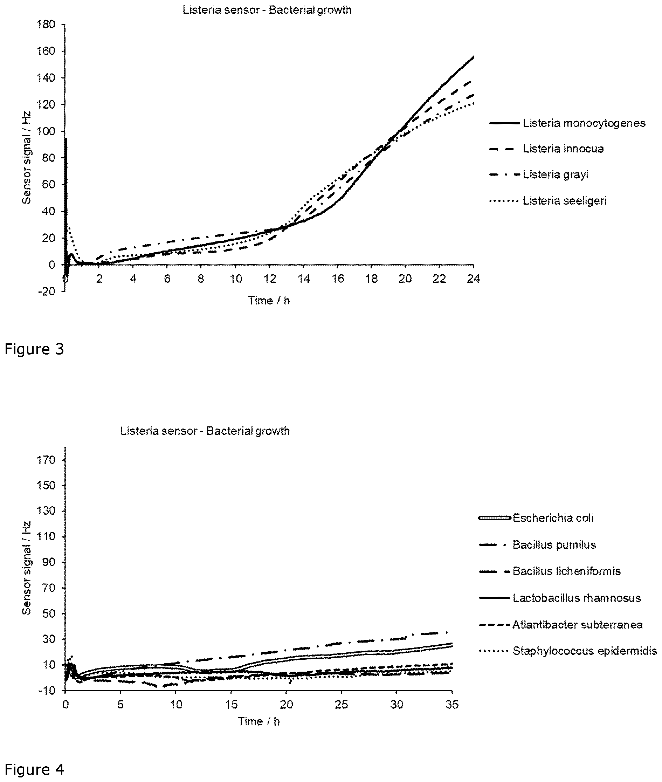

Figure 3 shows an example for the detection of viable *Listeria* species. In this example *Listeria monocytogenes, Listeria innocua, Listeria grayi* und *Listeria seeligeri,* all at a concentration of $10^5$ CFU/mL in the growth medium were detected within 12 to 16 h after addition in independent experiments. The sensors were prepared according to the aforementioned procedure with protein A coupled to the linker. The polyclonal antibody (2) was used as detection element at a concentration of 20 $\mu$g/mL in the cell medium. The measurement was performed as described above. The microorganisms were added by pipetting 50 $\mu$L of $10^6$ CFU/mL bacteria (range estimated by optical density measurements) in growth medium directly into 450 $\mu$L growth medium in the measuring cell, again resulting in a concentration of $10^5$ CFU/mL of the bacteria in the growth medium.

The same measurement setup and sensors were used in the experiments shown in Figure 4, where six different species (*Escherichia coli, Bacillus pumilus, Bacillus, licheniformis, Lactobacillus rhamnosus, Atlantibacter subterranea,* and *Staphylococcus epidermidis*) were cultivated and diluted to the same concentration ($10^5$ CFU/mL) and did not belong to the genus *Listeria.* The experiment shows that the six species did not lead to a sensor signal increase, which proves that the sensor setup was only selective for the genus *Listeria.*

Example 3: *Escherichia coli Sensor*

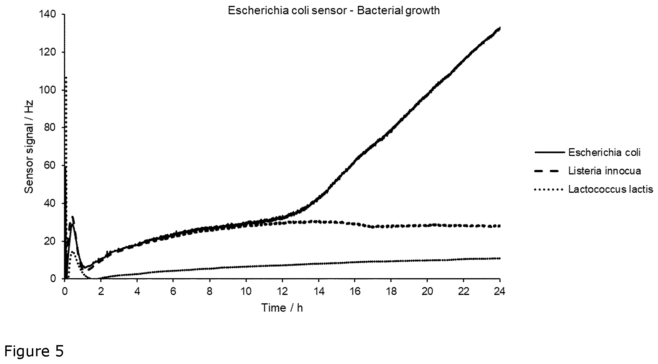

In this example a sensor was prepared according to the aforementioned procedure with protein A coupled to the linker. As detection element and signal amplifiers anti-*Escherichia coli* polyclonal antibodies (3) were used. The measurement was performed as described above, using a concentration of 40 $\mu$g/mL antibody in the cell medium. The microorganisms were added by pipetting 50 $\mu$L of bacteria solution (concentration range estimated by optical density measurements) in growth medium directly into 450 $\mu$L growth medium in the measuring cell. Figure 5 shows that *Escherichia coli* ($10^3$ CFU/mL in measuring cell) was detected in the cell medium as an increased sensor signal after about 14 hours of incubation. The species *Listeria innocua* ($10^2$ CFU/mL in measuring cell) and *Lactococcus lactis* ($10^5$ CFU/mL in measuring cell) did not cause an increased sensor signal, even after 24 hours of measurement. The sensor is therefore able to detect *Escherichia coli* and is specific for the detection of *Escherichia coli*. The example shows that the invention is generic and can be applied to different bacteria.

Example 4: Measurement of living and dead bacteria

In this example a sensor was prepared according to the aforementioned procedure with protein A coupled to the linker. As detection element and signal enhancer 20 $\mu$g/mL of an anti-Listeriolysin O antibody (1) was used. The measurement was performed as described above. The microorganisms were added by pipetting 50 $\mu$L of bacteria (dead or alive) solution ($10^3$ CFU/mL, concentration range determined by optical density measurements) in growth medium directly into 450 $\mu$L growth medium in the measuring cell. Dead bacteria were obtained by autoclaving (120 °C at 3 bar for 20 minutes) an aliquot of the living bacteria.

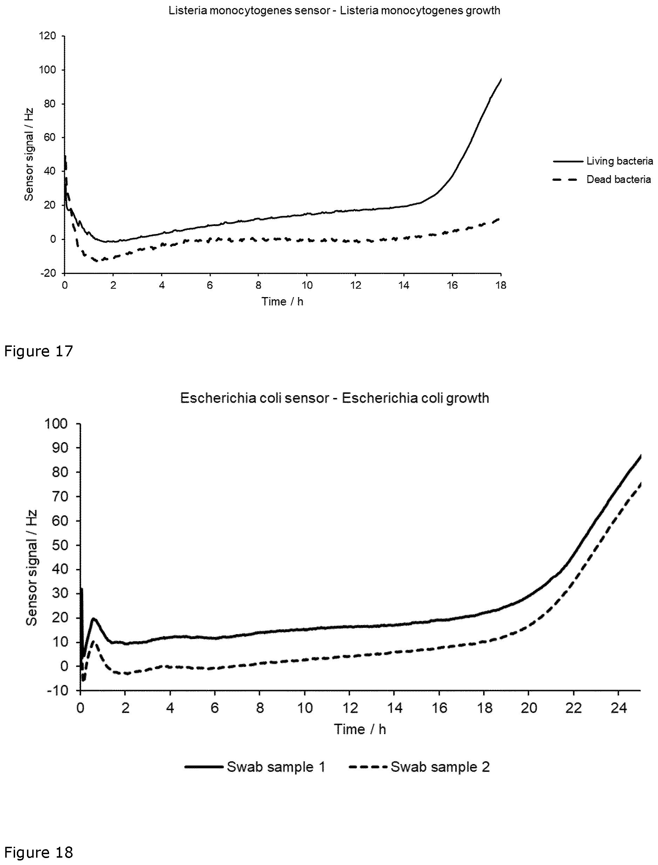

Figure 17 shows the measurement of living and dead *L. monocytogenes*. A sensor signal was only obtained when living bacteria were added to the cell medium, which proves their viability. As a control and to proof the viability of bacteria, dead and living bacteria were also plated on agar plates. After incubation at 37 °C, colonies were obtained only from living (non-autoclaved) bacteria. This proves that the measured samples were indeed living (colonies have formed) or dead (no colonies formed) bacteria, respectively. For comparison: Single PCR measurements cannot distinguish between dead and living bacteria.

Example 5

One-step procedure including enrichment and detection of swab samples.

In this example a sensor was prepared according to the aforementioned procedure with protein A coupled to the linker. As detection element and signal amplifier anti *Escherichia coli* polyclonal antibodies were used, i.e. antibody (3) as stated above under "materials". The measurement was performed as described above, using 20 $\mu$g/mL antibody.

In this experiment two swab samples were measured independently, both of which comprised 1 - 50 CFUs of *Escherichia coli* per swab (Th. Geyer GmbH&Co. KG, Renningen, Germany; Article number 80520CS). The swabs were spiked with 70 $\mu$L of bacteria solution with known concentration of $10^2$ CFU/mL. The swab samples were directly immersed in the cell medium (630 $\mu$L) inside the measuring cell and remained there for the whole measurement. The measurement was otherwise performed as described in Example 3. As can be seen from Figure 18, a positive signal was detected within about 20 hours.

This experiment shows that sample preparation is extremely simple with the inventive method. Taking the swab sample and inserting it directly into the measuring cell is sufficient. The measurement itself includes only one step, namely inserting the sample (here: swab sample) into the measuring cell. Known methods rely on at least one first enrichment step and often further steps followed by detection/determination of the target microorganism (e.g. PCR rapid tests), require more handling steps, additional equipment and are therefore much more difficult to handle in industrial practice.

Example 6

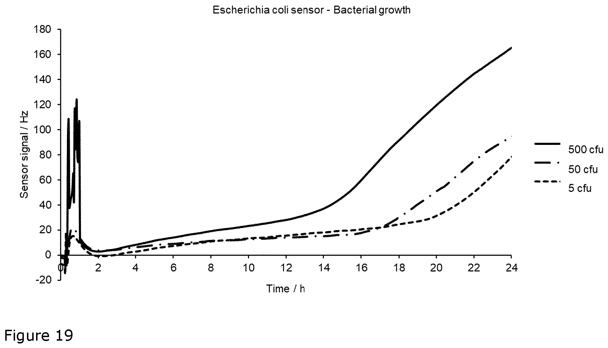

In this experiment Example 3 was repeated with three different concentrations (5 CFU, 50 CFU and 500 CFU) of *Escherichia coli* in the cell medium (concentration of an *Escherichia coli* stock solution was determined by plating on agar). As can be seen from Figure 19, the present method can measure concentrations of *Escherichia coli* up to 5 CFU and lower, reliably. It is evident that higher concentrations provide a signal at an earlier point in time.

*Comparative examples*

Comparative Example 1

In this example the influence of the buffer on selectivity is illustrated. For this purpose, a sensor was used that was not functionalized with a detection element and should therefore not show a signal when in contact with bacteria during incubation. Two kind of sensors were prepared. The cleaning step was the same as described above. The first kind of sensors were only functionalized with a self-assembled monolayer consisting of 30 mM 1-octanthiol (OT) in ethanol for 3 hours, while the other kind of sensors were only functionalized with a self-assembled monolayer consisting of 11-mercapto-1-undecanol (MCU), 30 mM in ethanol for 3 hours. These two types of sensors represent functionalized surfaces that are either hydrophobic (1-octanethiol, OT) or hydrophilic (11-mercapto-1-undecanol, MCU). One set of measurements was carried out with tryptic soy broth (TSB) as growth medium and another set of measurements was carried out with medium that has been buffered additionally. The buffered medium was prepared by mixing 1:1 TSB and 10xPBS vol%. 800 $\mu$L of growth medium or buffered growth medium was added to the measuring cell and the system equilibrated to 37°C until a stable baseline was reached. Subsequently, 25 $\mu$L bacteria solution was added directly into the growth medium in the measuring cell. For measurements in growth medium the concentration of the bacteria solution was $2.7 \times 10^5$ CFU/mL, while measurements in buffered growth medium were made at a concentration of $5.0 \times 10^5$ CFU/mL leading to bacteria concentrations in the measuring cell in the range of $10^3$ - $10^4$ CFU/mL. The cell medium was incubated at 37°C for 18 hours.

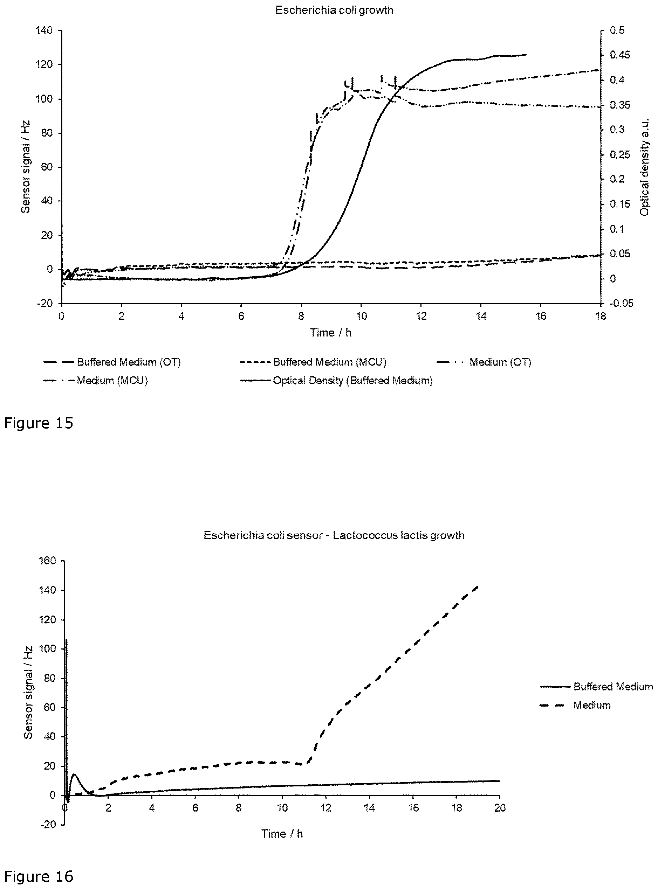

Two experiments (one sensor OT and one sensor MCU) were carried out with buffered medium (TSB/10$\times$PBS) and two experiments (one sensor OT and one sensor MCU) with only TSB as growth medium. As can be seen from Figure 15, the sensor shows a signal increase after about 8 hours for the two experiments that were conducted in growth medium without buffer. This is unwanted, as the sensor is not equipped with a detection element. The bacteria should not bind to the sensor surface. However, measurements in buffered medium show that no signal increase can be seen even after 18 hours (as intended/expected) due to the fact that no detection element is present on the sensor surface. To confirm that bacterial growth took place in the buffered medium, the optical density (monitored at 37°C, 610 nm taking a measurement point every 30 minutes) of the cell medium was measured (solid line). As can be seen, after about 8 hours the readout of the optical density confirms the bacterial growth. Furthermore, bacterial growth was confirmed by plating the cell medium after measurements and determining the concentration.

It could be proven that the signal obtained with the unmodified cell medium was caused by unspecific binding of cell medium ingredients (e.g. proteins) due to major pH changes. The experiments show that buffering the cell medium impedes unspecific deposition of material on the sensor and does not result in a sensor response as no binding to or deposition on the sensor surface takes place. The sensor signal in the cell medium that is not buffered is a result of unspecific binding or deposition as a result of major pH changes of the cell medium. The buffering of the cell medium minimizes or eliminates unspecific binding of ingredients in the cell medium to the sensor that are a result of major pH changes.

Comparative example 2

In this experiment a sensor was prepared with an anti-*Escherichia coli* antibody as in Example 3 and the measurement was performed with a cell medium that comprised *L. lactis.* The microorganisms were added by pipetting 50 $\mu$L of bacteria solution (concentration range determined by optical density measurements) in growth medium directly into 450 $\mu$L growth medium in the measuring cell. Two experiments were performed: one with additional buffered growth medium (*L. lactis* concentration in measuring cell $10^5$ CFU/mL) and one without additional buffered growth medium (*L. lactis* concentration in measuring cell $10^3$ CFU/mL), both as described in "Comparative example 1". Figure 16 shows a signal increase after about 11 hours in the case of using a cell medium that was not buffered (dashed line), even though *L. lactis* was not expected to cause sensor signals, as the detection elements and signal amplifiers were selective for *Escherichia coli.* In contrast, in the experiment containing buffered cell medium no signal was recorded. This example underlines the role of the buffer during microorganism growth in complex biological media.

**Claims**

1. Measuring device for the detection of one or more microorganisms, the measuring device comprising a measuring cell, one or more sensors and a cell medium, wherein

   - the measuring cell comprises the one or more sensors and the cell medium,
   - one or more detection elements are immobilized on at least one part of a surface of each of the one or more sensors,
   - the one or more detection elements are in contact with the cell medium,
   - the at least one or more detection elements comprise one or more binding sites that selectively bind to one or more analytes, wherein the one or more analytes may be any substance that is an indicator for the presence of the one or more microorganisms,

- the one or more sensors are able to detect the binding of at least one of the one or more analytes to the one or more sensors,

**characterized in that**

- the cell medium is a culture medium that supports the growth of the microorganisms to be detected and **in that**
- the measuring device comprises means to control the pH value of the cell medium.

2. Method for the detection of one or more microorganisms comprising the following steps:

- providing a measuring device that comprises a measuring cell that comprises one or more sensors, wherein

- one or more detection elements are immobilized on at least one part of a surface of each of the one or more sensors and
- the one or more detection elements comprise at least one compound that selectively binds to at least one of one or more analytes, wherein the one or more analytes may be any substance that is an indicator for the presence of the one or more microorganisms and the one or more sensors are able to detect the binding of the at least one of the one or more analytes to the sensor,

- introducing a cell medium into the measuring cell,
- at least one part of a surface of each of the one or more sensors on which the one or more detection elements are immobilized is brought into contact with the cell medium, and
- introducing one or more analytes into the cell,

**characterized in that**

- the cell medium is a culture medium that supports the growth of the one or more microorganisms to be detected
- the pH value of the cell medium is controlled throughout the measurement.

3. The measuring device of claim 1 or the method of claim 2, wherein the pH value of the cell medium is controlled within a range of 2 pH value units.

4. The measuring device of claim 1 or the method of claim 2, wherein the cell medium comprises a buffer.

5. The measuring device or the method of any of the preceding claims, wherein the measuring device comprises means for continuous detection.

6. The method of any of the preceding claims, wherein the detection is continuous.

7. The measuring device or the method of any of the preceding claims, wherein at least one of the one or more sensors is a quartz crystal microbalance.

8. The measuring device or the method of any of the preceding claims, wherein the surface of the sensor is coated with gold.

9. The measuring device or the method of any of the preceding claims, wherein at least one of the one or more sensor has one or more linker that binds at least one of the one or more detection elements to the sensor.

10. The measuring device or the method of any of the preceding claims, wherein the at least one of the one or more detection elements is selected from the group consisting of proteins, DNA, RNA, antibodies or parts thereof, aptamers, lectins, metal organic frameworks, drugs, toxins, polymers and cells.

11. The measuring device or the method of any of the preceding claims, wherein the detection element is an antibody.

12. The measuring device or the method of any of the preceding claims, wherein the cell medium comprises a signal amplifier.

13. The measuring device or the method of any of the preceding claims, wherein at least one of the one or more analytes is selected from the group consisting of *Escherichia coli, Salmonella Typhimurium, Listeria monocytogenes, Bacillus cereus,* Pseudomonas and *Francisella tularensis.*

14. The measuring device or the method of any of the preceding claims, wherein the culture medium is a selective culture medium that selectively supports the growth of at least one of the one or more microorganisms to be detected.

15. The measuring device or the method of any of the preceding claims wherein the detection is a quantitative measurement of at least one of the one or more microorganisms.

16. The measuring device or the method of any of the preceding claims wherein the analyte is a pathogen.

Figure 1

Figure 2

Listeria sensor - Bacterial growth

Figure 3

Listeria sensor - Bacterial growth

Figure 4

Figure 5

Figure 6

Figure 7

Figure 8

Figure 9

Figure 10

Figure 11

Figure 12

Figure 13

Figure 14

Figure 15

Figure 16

Listeria monocytogenes sensor - Listeria monocytogenes growth

Figure 17

Escherichia coli sensor - Escherichia coli growth

Figure 18

Escherichia coli sensor - Bacterial growth

Figure 19

Europäisches
Patentamt

European
Patent Office

Office européen
des brevets

## EUROPEAN SEARCH REPORT

**Application Number**

EP 24 22 3788

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XIAO GUO ET AL: "A piezoelectric immunosensor for specific capture and enrichment of viable pathogens by quartz crystal microbalance sensor, followed by detection with antibody-functionalized gold nanoparticles", BIOSENSORS AND BIOELECTRONICS, ELSEVIER SCIENCE LTD, UK, AMSTERDAM , NL, vol. 38, no. 1, 18 May 2012 (2012-05-18), pages 177-183, XP028398342, ISSN: 0956-5663, DOI: 10.1016/J.BIOS.2012.05.024 [retrieved on 2012-05-29] * Abstract, Results, Figures 1 and 3A * | 1-16 | INV. G01N33/543 G01N33/569 |
| X | LATIF USMAN ET AL: "Biomimetic Receptors for Bioanalyte Detection by Quartz Crystal Microbalances - From Molecules to Cells", SENSORS, vol. 14, no. 12, 5 December 2014 (2014-12-05), pages 23419-23438, XP093277159, CH ISSN: 1424-8220, DOI: 10.3390/s141223419 Retrieved from the Internet: URL:https://www.mdpi.com/1424-8220/14/12/23419/pdf> * Abstract, Figure 11, Section 3.3 * | 1-16 | |

-----

-----

-/--

TECHNICAL FIELDS
SEARCHED      (IPC)

G01N

The present search report has been drawn up for all claims

| Place of search | Date of completion of the search | Examiner |
|---|---|---|
| Munich | 13 October 2025 | Behrens, Ralf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 22 3788

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | VIDOVIC SINISA ET AL: "Lifestyle of Listeria monocytogenes and food safety: Emerging listericidal technologies in the food industry", CRITICAL REVIEWS IN FOOD SCIENCE AND NUTRITION, vol. 64, no. 7, 5 September 2022 (2022-09-05), pages 1817-1835, XP093322626, USA ISSN: 1040-8398, DOI: 10.1080/10408398.2022.2119205 ----- | 1-16 | |
| | | | **TECHNICAL FIELDS SEARCHED (IPC)** |

The present search report has been drawn up for all claims

| Place of search | Date of completion of the search | Examiner |
|---|---|---|
| Munich | 13 October 2025 | Behrens, Ralf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☒ Only part of the further search fees have been paid within the fixed time limit. The present European search report has been drawn up for those parts of the European patent application which relate to the inventions in respect of which search fees have been paid, namely claims:

1-16(partially)

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

41

Europäisches
Patentamt

European
Patent Office

Office européen
des brevets

**LACK OF UNITY OF INVENTION**
**SHEET B**

Application Number

EP 24 22 3788

The Search Division considers that the present European patent application does not comply with the requirements of unity of invention and relates to several inventions or groups of inventions, namely:

    1. claims: 1-16(partially)

        System of claim 1, wherein the measuring device comprises
        means for continuous detection, the sensor is a gold QCM,
        the detection element is an antibody, the cell medium
        comprises a signal amplifier, and the analyte is Escherichia
        coli. Method of using the system, wherein the detection is
        continuous.
                            ---

    2. claims: 1-16(partially)

        System of claim 1, wherein the measuring device comprises
        means for continuous detection, the sensor is a gold QCM,
        the detection element is an antibody, the cell medium
        comprises a signal amplifier, the analyte is Listeria
        monocytogenes. Method of using the system, wherein the
        detection is continuous.
                            ---

    3. claims: 1-16(partially)

        Other systems and methods according to claims 1-2.
                            ---

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **A. SHONS** ; **F. DORMAN** ; **J. NAJARIAN**. *J. Biomed. Mater. Res.*, vol. 6, 565
- **G.A. CAMPBELL** ; **R. MUTHARASAN**. A method of measuring Escherichia coli 0157:H7 at 1 cell/mL in 1 liter sample using antibody functionalized piezo-electric-excited millimeter-sized cantilever sensor. *Environmental Science & Technology*, 2007, vol. 41, 1668-1674
- **X.-L. SU** ; **Y. LI**. A QCM immunosensor for Salmonella detection with simultaneous measurements of resonant frequency and motional resistance. *Biosensors and Bioelectronics*, 2005, vol. 21, 840-848, https://doi.org/10.1016/j.bios.2005.01.021
- **X.-L. SU et al.** A self-assembled monolayer-based piezoelectric immunosensor for rapid detection of Escherichia coli O157:H7. *Biosensors and Bioelectronics*, 2004, vol. 19, 563-574, https://doi.org/10.1016/S0956-5663(03)00254-9

- **R. RIPA et al.** Detecting Escherichia coli Biofilm Development Stages on Gold and Titanium by Quartz Crystal Microbalance. *ACS Omega*, 2020, vol. 5, 2295-2302, https://doi.org/10.1021/acsome-ga.9b03540
- **VAUGHAN et al.** Development of a quartz crystal microbalance (QCM) immunosensor for the detection of Listeria monocytogenes. *Enzyme and Microbial Technology*, 2001, vol. 29, 635-638, https://doi.org/10.1016/S0141-0229(01)00449-5
- **X. JIANG et al.** Evaluation of different micro/nanobeads used as signal amplifiers in QCM immunosensor for more sensitive detection of E. coli O157:H7. *Biosens. Bioelectron*, 2011, vol. 29, 23-28, https://doi.org/10.1016/j.bios.2011.07.059
- Fast detection of pathogenic bacteria by using different sensor techniques. **K. KLEO et al.** Proceedings IMCS 2012. AMA Service GmbH, 834-836
- Model BD 115. Binder GmbH